# FASCICULE DE BREVET EUROPEEN

(11) **EP 2 079 482 B1**
(45) Date de publication et mention de la délivrance du brevet: **23.02.2011**
(21) Numéro de dépôt: 07858450.5
(22) Date de dépôt: 15.10.2007
(51) Int. Cl.: C07K 16/28

(54) **UTILISATION D'UN ANTICORPS ANTI-CD151 POUR LE TRAITEMENT DE CANCER PRIMAIRE**
VERWENDUNG EINES ANTIKÖRPERS GEGEN CD151 BEI DER BEHANDLUNG VON PRIMÄREN KREBS
USE OF AN ANTI-CD151 ANTIBODY IN THE TREATMENT OF PRIMARY CANCER

(30) Priorité: 18.10.2006 FR 0609135
(43) Date de publication de la demande: 22.07.2009
(73) Titulaire: Pierre Fabre Medicament, 92100 Boulogne-Billancourt (FR)
(72) Inventeur: HAEUW, Jean-François, 74160 Beaumont (FR); GOETSCH, Liliane, 74130 Ayze (FR)
(74) Mandataire: Rousseau, Pierrick Edouard
(86) Numéro de dépôt international: PCT/FR2007/001688
(87) Numéro de publication internationale: WO 2008/049990

(56) Documents cités:
- WO-A-99/66027
- TESTA J E ET AL: "EUKARYOTIC EXPRESSION CLONING WITH AN ANTIMETASTATIC MONOCLONAL ANTIBODY IDENTIFIES A TETRASPANIN (PETA-3/CD151) AS AN EFFECTOR OF HUMAN TUMOR CELL MIGRATION AND METASTASIS" CANCER RESEARCH, AMERICAN ASSOCIATION FOR CANCER RESEARCH, BALTIMORE, MD, US, vol. 59, no. 15, 1 août 1999 (1999-08-01), pages 3812-3820, XP008064694 ISSN: 0008-5472 cité dans la demande
- SERRU V ET AL: "Selective tetraspan-integrin complexes (CD81/alpha4beta1, CD151/alpha3beta1, CD151/alpha6beta1) under conditions disrupting tetraspan interactions." THE BIOCHEMICAL JOURNAL 15 MAY 1999, vol. 340 ( Pt 1), 15 mai 1999 (1999-05-15), pages 103-111, XP002435969 ISSN: 0264-6021 cité dans la demande
- GEARY S M ET AL: "Differential tissue expression of epitopes of the tetraspanin CD151 recognised by monoclonal antibodies." TISSUE ANTIGENS SEP 2001, vol. 58, no. 3, septembre 2001 (2001-09), pages 141-153, XP002435970 ISSN: 0001-2815 cité dans la demande
- CHOMETON ET AL: "Dissociation of the complex between CD151 and laminin-binding integrins permits migration of epithelial cells" EXPERIMENTAL CELL RESEARCH, SAN DIEGO, CA, US, vol. 312, no. 7, 15 avril 2006 (2006-04-15), pages 983-995, XP005346073 ISSN: 0014-4827 cité dans la demande
- ZIJLSTRA ANDRIES ET AL: "The inhibition of tumor cell intravasation and subsequent metastasis via regulation of in vivo tumor cell motility by the tetraspanin CD151" CANCER CELL, vol. 13, no. 3, mars 2008 (2008-03), pages 221-234, XP002487814 ISSN: 1535-6108

### LISTE DE SEQUENCES

<110> Pierre Fabre Médicament
<120> utilisation d'un anticorps anti-CD151 pour le traitement du cancer
<130> cas 640
<150> FR 06/09135
   <151> 2006-10-18
<160> 38
<170> PatentIn version 3.3
<210> 1
   <211> 759
   <212> DNA
   <213> Homo sapiens
<400> 1
   atgggtgagt tcaacgagaa gaagacaaca tgtggcaccg tttgcctcaa gtacctgctg     60
   tttacctaca attgctgctt ctggctggct ggcctggctg tcatggcagt gggcatctgg    120
   acgctggccc tcaagagtga ctacatcagc ctgctggcct caggcaccta cctggccaca    180
   gcctacatcc tggtggtggc gggcactgtc gtcatggtga ctggggtctt gggctgctgc    240
   gccaccttca aggagcgtcg gaacctgctg cgcctgtact tcatcctgct cctcatcatc    300
   tttctgctgg agatcatcgc tggtatcctc gcctacgcct actaccagca gctgaacacg    360
   gagctcaagg agaacctgaa ggacaccatg accaagcgct accaccagcc gggccatgag    420
   gctgtgacca gcgctgtgga ccagctgcag caggagttcc actgctgtgg cagcaacaac    480
   tcacaggact ggcgagacag tgagtggatc cgctcacagg aggccggtgg ccgtgtggtc    540
   ccagacagct gctgcaagac ggtggtggct ctttgtgggc agcgagacca tgcctccaac    600
   atctacaagg tggagggcgg ctgcatcacc aagttggaga ccttcatcca ggagcacctg    660
   agggtcattg gggctgtggg gatcggcatt gcctgtgtgc aggtctttgg catgatcttc    720
   acgtgctgcc tgtacaggag tctcaagctg gagcactac                           759
<210> 2
   <211> 253
   <212> PRT
   <213> homo sapiens
<400> 2
   Met Gly Glu Phe Asn Glu Lys Lys Thr Thr Cys Gly Thr Val Cys Leu
   1               5                   10                  15
   Lys Tyr Leu Leu Phe Thr Tyr Asn Cys Cys Phe Trp Leu Ala Gly Leu
               20                  25                  30
   Ala Val Met Ala Val Gly Ile Trp Thr Leu Ala Leu Lys Ser Asp Tyr
           35                  40                  45
   Ile Ser Leu Leu Ala Ser Gly Thr Tyr Leu Ala Thr Ala Tyr Ile Leu
   50 55 60
   Val Val Ala Gly Thr Val Val Met Val Thr Gly Val Leu Gly Cys Cys
   65 70 75 80
   Ala Thr Phe Lys Glu Arg Arg Asn Leu Leu Arg Leu Tyr Phe Ile Leu
   85 90 95
   Leu Leu Ile Ile Phe Leu Leu Glu Ile Ile Ala Gly Ile Leu Ala Tyr
   100 105 110
   Ala Tyr Tyr Gln Gln Leu Asn Thr Glu Leu Lys Glu Asn Leu Lys Asp
   115 120 125
   Thr Met Thr Lys Arg Tyr His Gln Pro Gly His Glu Ala Val Thr Ser
   130 135 140
   Ala Val Asp Gln Leu Gln Gln Glu Phe His Cys Cys Gly Ser Asn Asn
   145 150 155 160
   Ser Gln Asp Trp Arg Asp Ser Glu Trp Ile Arg Ser Gln Glu Ala Gly
   165 170 175
   Gly Arg Val Val Pro Asp Ser Cys Cys Lys Thr Val Val Ala Leu Cys
   180 185 190
   Gly Gln Arg Asp His Ala Ser Asn Ile Tyr Lys Val Glu Gly Gly Cys
   195 200 205
   Ile Thr Lys Leu Glu Thr Phe Ile Gln Glu His Leu Arg Val Ile Gly
   210 215 220
   Ala Val Gly Ile Gly Ile Ala Cys Val Gln Val Phe Gly Met Ile Phe
   225 230 235 240
   Thr Cys Cys Leu Tyr Arg Ser Leu Lys Leu Glu His Tyr
   245 250
<210> 3
   <211> 327
   <212> DNA
   <213> homo sapiens
<400> 3
   gcctactacc agcagctgaa cacggagctc aaggagaacc tgaaggacac catgaccaag 60
   cgctaccacc agccgggcca tgaggctgtg accagcgctg tggaccagct gcagcaggag 120
   ttccactgct gtggcagcaa caactcacag gactggcgag acagtgagtg gatccgctca 180
   caggaggccg gtggccgtgt ggtcccagac agctgctgca agacggtggt ggctctttgt 240
   gggcagcgag accatgcctc caacatctac aaggtggagg gcggctgcat caccaagttg 300
   gagaccttca tccaggagca cctgagg 327
<210> 4
   <211> 109
   <212> PRT
   <213> hoo sapiens
<400> 4
   Ala Tyr Tyr Gln Gln Leu Asn Thr Glu Leu Lys Glu Asn Leu Lys Asp
   1 5 10 15
   Thr Met Thr Lys Arg Tyr His Gln Pro Gly His Glu Ala Val Thr Ser
   20 25 30
   Ala Val Asp Gln Leu Gln Gln Glu Phe His Cys Cys Gly Ser Asn Asn
   35 40 45
   Ser Gln Asp Trp Arg Asp Ser Glu Trp Ile Arg Ser Gln Glu Ala Gly
   50 55 60
   Gly Arg Val Val Pro Asp Ser Cys Cys Lys Thr Val Val Ala Leu Cys
   65 70 75 80
   Gly Gln Arg Asp His Ala Ser Asn Ile Tyr Lys Val Glu Gly Gly Cys
   85 90 95
   Ile Thr Lys Leu Glu Thr Phe Ile Gln Glu His Leu Arg
   100 105
<210> 5
   <211> 54
   <212> DNA
   <213> homo sapiens
<400> 5
   tggacgctgg ccctcaagag tgactacatc agcctgctgg cctcaggcac ctac 54
<210> 6
   <211> 18
   <212> PRT
   <213> hoo sapiens
<400> 6
   Trp Thr Leu Ala Leu Lys Ser Asp Tyr Ile Ser Leu Leu Ala Ser Gly
   1               5                   10                  15
   Thr Tyr
<210> 7
   <211> 8
   <212> PRT
   <213> mus musculus
<400> 7
   Gly Tyr Thr Phe Thr Asp Tyr Ser
   1               5
<210> 8
   <211> 8
   <212> PRT
   <213> mus musculus
<400> 8
   Ile Asn Thr Glu Thr Gly Glu Pro
   1               5
<210> 9
   <211> 13
   <212> PRT
   <213> mus musculus
<400> 9
   Ala Arg Arg Glu Tyr Gly Asn Tyr Tyr Gly Met Glu Tyr
   1               5                   10
<210> 10
   <211> 120
   <212> PRT
   <213> mus musculus
<400> 10
   Gln Ile Gln Leu Val Gln Ser Gly Pro Glu Leu Lys Lys Pro Gly Glu
   1               5                   10                  15
   Thr Val Lys Ile Ser Cys Lys Ala Ser Gly Tyr Thr Phe Thr Asp Tyr
               20                  25                  30
   Ser Met His Trp Val Lys Gln Ala Pro Gly Lys Gly Leu Lys Trp Met
           35                  40                  45
   Gly Trp Ile Asn Thr Glu Thr Gly Glu Pro Thr Tyr Thr Asp Asp Phe
       50                  55                  60
   Lys Gly Arg Phe Ala Phe Ser Leu Glu Thr Ser Ala Asn Thr Ala Tyr
   65                  70                  75                  80
   Leu Arg Ile Asn Asn Leu Lys Asn Glu Asp Thr Ala Thr Tyr Phe Cys
                   85                  90                  95
   Ala Arg Arg Glu Tyr Gly Asn Tyr Tyr Gly Met Glu Tyr Trp Gly Gln
               100                 105                 110
   Gly Thr Ser Val Thr Val Ser Ser
           115                 120
<210> 11
   <211> 10
   <212> PRT
   <213> mus musculus
<400> 11
   Gln Ser Val Ser Thr Ser Thr Phe Ser Tyr
   1               5                   10
<210> 12
   <211> 3
   <212> PRT
   <213> mus musculus
<400> 12
   Ser Ala Ser
   1
<210> 13
   <211> 9
   <212> PRT
   <213> mus musculus
<400> 13
   Gln His Ser Trp Glu Ile Pro Leu Thr
   1               5
<210> 14
   <211> 111
   <212> PRT
   <213> mus musculus
<400> 14
   Asp Ile Val Leu Thr Gln Ser Pro Ala Ser Leu Ala Val Ser Leu Gly
   1               5                   10                  15
   Gln Arg Ala Thr Ile Ser Cys Arg Ala Ser Gln Ser Val Ser Thr Ser
               20                  25                  30
   Thr Phe Ser Tyr Ile His Trp Tyr Gln Gln Lys Pro Gly Gln Pro Pro
           35                  40                  45
   Lys Leu Leu Ile Lys Ser Ala Ser Asn Leu Glu Ser Gly Val Pro Ala
       50                  55                  60
   Arg Phe Ser Gly Ser Gly Ser Gly Thr Asp Phe Thr Leu Thr Ile His
   65                  70                  75                  80
   Pro Val Glu Glu Glu Asp Thr Ala Thr Tyr Phe Cys Gln His Ser Trp
                   85                  90                  95
   Glu Ile Pro Leu Thr Phe Gly Ala Gly Thr Lys Leu Glu Leu Lys
               100                 105                 110
<210> 15
   <211> 8
   <212> PRT
   <213> mus musculus
<400> 15
   Gly Tyr Thr Phe Thr Ser Ser Trp
   1               5
<210> 16
   <211> 8
   <212> PRT
   <213> mus musculus
<400> 16
   Ile His Pro Asn Ser Gly Asn Thr
   1 5
<210> 17
   <211> 16
   <212> PRT
   <213> mus musculus
<400> 17
   Ala Arg Gly Asp Asp Ala Tyr Tyr Ser Gly Leu Tyr Ala Met Asp Tyr
   1 5 10 15
<210> 18
   <211> 123
   <212> PRT
   <213> mus musculus
<400> 18
   Gln Val Gln Leu Gln Gln Pro Gly Ser Val Val Val Arg Pro Gly Ala
   1 5 10 15
   Ser Val Lys Leu Ser Cys Lys Ala Ser Gly Tyr Thr Phe Thr Ser Ser
   20 25 30
   Trp Met His Trp Ala Lys Gln Arg Pro Gly Gln Gly Leu Glu Trp Ile
   35 40 45
   Gly Gln Ile His Pro Asn Ser Gly Asn Thr Asn Tyr Asn Glu Lys Phe
   50 55 60
   Lys Val Lys Ala Thr Leu Thr Ile Asp Thr Ser Ser Ser Thr Ala Tyr
   65 70 75 80
   Val Asp Leu Asn Ser Leu Thr Ser Gly Asp Ser Ala Val Tyr Tyr Cys
   85 90 95
   Ala Arg Gly Asp Asp Ala Tyr Tyr Ser Gly Leu Tyr Ala Met Asp Tyr
   100 105 110
   Trp Gly Gln Gly Thr Ser Val Thr Val Ser Ser
   115 120
<210> 19
   <211> 10
   <212> PRT
   <213> mus musculus
<400> 19
   Gln Ser Val Ser Thr Ser Arg Tyr Ser Tyr
   1 5 10
<210> 20
   <211> 3
   <212> PRT
   <213> mus musculus
<400> 20
   Tyr Ala Ser
   1
<210> 21
   <211> 9
   <212> PRT
   <213> mus musculus
<400> 21
   Gln His Ser Trp Glu Ile Pro Tyr Thr
   1 5
<210> 22
   <211> 111
   <212> PRT
   <213> mus musculus
<400> 22
   Asp Ile Val Leu Thr Gln Ser Pro Ala Ser Leu Ala Val Ser Leu Gly
   1 5 10 15
   Gln Arg Ala Thr Ile Ser Cys Arg Ala Ser Gln Ser Val Ser Thr Ser
   20 25 30
   Arg Tyr Ser Tyr Met His Trp Tyr Gln Gln Ile Gln Gly Gln Pro Pro
   35 40 45
   Lys Leu Leu Ile Lys Tyr Ala Ser Asn Leu Glu Ser Gly Val Pro Ala
   50 55 60
   Arg Phe Ser Gly Ser Gly Ser Gly Thr Asp Phe Thr Leu Asn Ile His
   65 70 75 80
   Pro Val Glu Glu Glu Asp Thr Ala Thr Tyr His Cys Gln His Ser Trp
   85 90 95
   Glu Ile Pro Tyr Thr Phe Gly Gly Gly Thr Thr Leu Glu Ile Lys
   100 105 110
<210> 23
   <211> 24
   <212> DNA
   <213> mus musculus
<400> 23
   ggttatacct tcacagacta ttca 24
<210> 24
   <211> 24
   <212> DNA
   <213> mus musculus
<400> 24
   ataaacactg agactggtga gcca 24
<210> 25
   <211> 39
   <212> DNA
   <213> mus musculus
<400> 25
   gctagaaggg agtatggtaa ctactatggt atggagtac 39
<210> 26
   <211> 360
   <212> DNA
   <213> mus musculus
<400> 26
   cagatccagt tggtgcagtc tggacctgag ctgaagaagc ctggagagac agtcaagatc 60
   tcctgcaagg cttctggtta taccttcaca gactattcaa tgcactgggt gaagcaggct 120
   ccaggaaagg gtttaaagtg gatgggctgg ataaacactg agactggtga gccaacatat 180
   acagatgact tcaagggacg gtttgccttc tctttggaaa cctctgccaa cactgcctat 240
   ttgcggatca acaacctcaa aaatgaggac acggctacat atttctgtgc tagaagggag 300
   tatggtaact actatggtat ggagtactgg ggtcaaggaa cctcagtcac cgtctcgtca 360
<210> 27
   <211> 30
   <212> DNA
   <213> mus musculus
<400> 27
   caaagtgtca gtacatctac ctttagttat 30
<210> 28
   <211> 9
   <212> DNA
   <213> mus musculus
<400> 28
   tctgcatcc 9
<210> 29
   <211> 27
   <212> DNA
   <213> mus musculus
<400> 29
   cagcacagtt gggagattcc gctcacg 27
<210> 30
   <211> 333
   <212> DNA
   <213> mus musculus
<400> 30
   gacattgtgc tgacacagtc tcctgcttcc ttagctgtat ctctggggca gagggccacc 60
   atctcatgca gggccagcca aagtgtcagt acatctacct ttagttatat acactggtac 120
   caacagaaac caggacagcc acccaaactc ctcatcaagt ctgcatccaa cctagaatct 180
   ggggtccctg ccaggttcag tggcagtggg tctgggacag acttcaccct caccatccat 240
   cctgtggagg aggaggatac tgcaacatat ttctgtcagc acagttggga gattccgctc 300
   acgttcggtg ctgggaccaa gctggagctg aaa 333
<210> 31
   <211> 24
   <212> DNA
   <213> mus musculus
<400> 31
   ggctacacct tcaccagctc ctgg 24
<210> 32
   <211> 24
   <212> DNA
   <213> mus musculus
<400> 32
   attcatccta atagtggtaa tact 24
<210> 33
   <211> 48
   <212> DNA
   <213> mus musculus
<400> 33
   gcaagagggg atgatgctta ctacagcggg ctctatgcta tggactac 48
<210> 34
<211> 369
   <212> DNA
   <213> mus musculus
<400> 34
   caggtccaac tgcagcagcc tgggtctgtg gtggtgaggc ctggagcttc agtgaaactg 60
   tcctgcaagg cttctggcta caccttcacc agctcctgga tgcactgggc gaagcagagg 120
   cctggacaag gccttgagtg gattggacag attcatccta atagtggtaa tactaattac 180
   aatgagaagt tcaaggtcaa ggccacactg actatagaca catcctccag cacagcctac 240
   gtggatctca acagcctgac atctggggac tctgcggtct attactgtgc aagaggggat 300
   gatgcttact acagcgggct ctatgctatg gactactggg gtcagggaac ctcagtcacc 360
   gtctcctca 369
<210> 35
   <211> 30
   <212> DNA
   <213> mus musculus
<400> 35
   caaagtgtca gtacatctag gtatagttat 30
<210> 36
   <211> 9
   <212> DNA
   <213> mus musculus
<400> 36
   tatgcatcc 9
<210> 37
   <211> 27
   <212> DNA
   <213> mus musculus
<400> 37
   caacacagtt gggagattcc gtacacg 27
<210> 38
   <211> 333
   <212> DNA
   <213> mus musculus
<400> 38
   gacattgtgc tgacacagtc tcctgcttcc ttagctgtat ctctggggca gagggccacc 60
   atctcatgca gggccagcca aagtgtcagt acatctaggt atagttatat gcactggtac 120
   caacagatac aaggacagcc acccaaactc ctcatcaagt atgcatccaa cctagaatct 180
   ggggtccctg ccaggttcag tggcagtggg tctgggacag acttcaccct caacatccat 240
   cctgtggagg aggaggatac tgcaacatat cactgtcaac acagttggga gattccgtac 300
   acgttcggag gggggaccac gctggaaata aaa 333

## Description

La présente demande est relative à une nouvelle utilisation d'anticorps anti-CD151 capables d'inhiber la croissance tumorale, lesdits anticorps étant notamment monoclonaux d'origine murine, chimérique et humanisée. Selon un aspect particulier, la demande a pour objet l'utilisation de ces anticorps, ou de leurs fragments fonctionnels, à titre de médicament pour le traitement prophylactique et/ou thérapeutique des cancers. La demande comprend enfin des produits et/ou des compositions comprenant de tels anticorps en association, par exemple, avec des anticorps et/ou des agents anticancéreux ou conjugués avec des toxines et leur utilisation pour la prévention et/ou le traitement de certains cancers.

CD151, appelé aussi PETA-3 ou SFA-1, est une protéine membranaire appartenant à la famille des tétraspanines (Boucheix et Rubinstein, 2001, Cell Mol. Life Sci. 58, 1189-1205 ; Hemler, 2001, J. Cell Biol. 155, 1103-1107). Chez l'homme, CD151 possède 253 acides aminés, et comporte 4 fragments membranaires et 2 domaines extracellulaires EC1 (18 acides aminés, séquence [40-57]) et EC2 (109 acides aminés, séquence [113-221]), appelés aussi boucles extracellulaires. Il est cependant à noter qu'au niveau de la séquence nucléotidique, deux variants pour CD151 ont été identifiés à ce jour, à savoir l'un comprenant les nucléotides A et C respectivement aux positions 395 et 409 (SEQ ID No. 1) [Fitter et al., 1995, Blood 86(4), 1348-1355] et l'autre comprenant pour les mêmes positions les nucléotides G et T au lieu et place des nucléotides A et C [Hasegawa et al., 1996, J. Virol. 70(5), 3258-3263]. De ce fait, une mutation au niveau de la séquence peptidique peut être observée, à savoir une mutation des résidus K (Lys) et P (Pro) respectivement aux positions 132 et 137 en les résidus R Arg) et S (Ser) [Fitter et al., 1995, Blood 86(4), 1348-1355 / Hasegawa et al., 1996, J. Virol. 70(5), 3258-3263].

**CD151** est surexprimé dans de nombreux cancers, comme par exemple les cancers du poumon [Tokuhara et al., 2001, Clin. Cancer Res. 7, 4109-4114], du colon [Hashida et al., 2003, Br. J. Cancer 89, 158-167], de la prostate [Ang et al., 2004, Cancer Epidemiol. Biomarkers Prev. 13, 1717-1721] ou du pancréas [Gesierich et al., 2005, Clin. Cancer Res. 11, 2840-2852].

L'utilisation des souris knock-out n'exprimant pas CD151, et d'anticorps anti-CD151 et de siRNA pour bloquer *in vitro* la fonctionnalité et l'expression de CD151 dans différentes types cellulaires a permis de montrer que CD151 est impliqué dans plusieurs phénomènes liés au cancer, comme l'adhésion cellulaire (Nishiuchi et al., 2005, Proc. Natl. Acad. Sci. USA 102, 1939-1944 ; Winterwood et al., 2006, Mol. Biol. Cell 17, 2707-2721), la motilité cellulaire (Kohno et al., 2002, Int. J. Cancer 97, 336-343), la migration cellulaire (Yauch et al., 1998, Mol. Biol. Cell 9, 2751-2765 ; Testa et al., 1999, Cancer Res. 59, 3812-3820 ; Penas et al., 2000, J. Invest. Dermatol. 114, 1126-1135 ; Klosek et al., 2005, Biochem. Biophys. Res. Commun. 336, 408-416), l'invasion cellulaire (Kohno et al., 2002, Int. J. Cancer 97, 336-343 ; Shiomi et al., 2005, Lab. Invest. 85, 1489-1506 ; Hong et al., 2006, J. Biol. Chem. 281, 24279-24292) et l'angiogénèse *(*Yanez-Mo et al., 1998, J. Cell Biol. 141, 791-804 ; Sincock et al., 1999, J. Cell Sci. 112, 833-844 ; Takeda et al., 2006, Blood).

L'une des propriétés remarquable des tétraspanines est leur capacité à s'associer entre elles, ainsi qu'à un nombre important d'autres molécules de surface pour former des complexes macromoléculaires structurés. Au sein de ces complexes, chaque tétraspanine est associée spécifiquement à une ou plusieurs molécules de surface formant ainsi des complexes primaires, constitués d'une tétraspanine et d'une molécule partenaire. Les tétraspanines peuvent organiser des microdomaines particuliers de la membrane plasmique au sein desquels elles recruteraient leurs partenaires moléculaires qui pourraient être couplés fonctionnellement. L'ensemble des interactions impliquant les tétraspanines a été dénommé « réseau des tétraspanines » ou « Tetraspanin Web ».

CD151 interagit à la surface des cellules avec différentes protéines membranaires. Des complexes très stables, résistants à l'action de certains détergents, ont notamment été mis en évidence avec les intégrines récepteurs des laminines, et plus particulièrement avec les intégrines α31β1 ou α6β4 dont le ligand préférentiel est la laminine 5 (Yauch et al., 1998, Mol. Biol. Cell 9, 2751-2765 ; Lammerding et al., 2003, Proc. Natl. Acad. Sci USA 100, 7616-7621). Cette association implique les domaines extracellulaires de CD151 et des intégrines. La séquence QRD [194-196] de CD151, localisée dans la boucle EC2, est très importante dans cette association puisque la mutation de ce site provoque la perte de l'interaction avec certaines intégrines (Kazarov et al., 2002, J. Cell Biol. 158, 1299-1309). Des complexes ternaires fonctionnels CD151/intégrine α6β4/c-Met (récepteur du HGF) ont d'autre part été mis en évidence dans les cellules tumorales (Klosek et al., 2005, Biochem. Biophys. Res. Commun. 336, 408-416). L'inhibition de l'expression de CD151 par traitement des cellules avec un ARN interférence provoque une inhibition de la croissance et de la migration cellulaire induite par HGF.

Les interactions au sein d'un même cellule de CD151 avec d'autres tétraspanines, nécessaires à la formation du réseau des tétraspanines, dépendraient des régions membranaires et cytoplasmiques de CD151 puisqu'il a été montré que la délétion de la boucle EC2 ne rompait pas l'association de CD151 avec d'autres tétraspanines (Berditchevski, 2001, J. Cell Sci. 114, 4143-4151).

CD151 est capable de réguler les phénomènes d'adhésion, de migration et d'invasion cellulaire par la modulation de différentes voies de signalisation, comme par exemple la voie des phosphoinositides via une association avec la PI4-kinase (Yauch et al., 1998, Mol. Biol. Cell 9, 2751-2765), la voie de signalisation de c-Jun via la phosphorylation de FAK, Src, p38-MAPK et JNK (Hong *et al.,* 2006), la phosphorylation des intégrines par la PKC (Zhang et al., 2001, J. Biol. Chem. 276, 25005-25013), l'activation de GTPases de la famille Rho (Shigeta et al., 2003, J. Cell Biol. 163, 165-176).

Des interactions de type homophilique entre cellules sont aussi responsables d'une augmentation de la motilité cellulaire et de l'expression de la métalloprotéinase MMP-9 (Hong *et al.,* 2006). Ces interactions intercellulaires CD151-CD151 provoquent l'activation de c-Jun via la phosphorylation de FAK, Src, p38-MAPK et JNK

A ce jour, en dépit de l'intérêt de la protéine CD151, seul un anticorps à visée thérapeutique a été généré, à savoir l'anticorps monoclonal 50-6.

L'anticorps monoclonal 50-6 (isotype IgG1) dirigé contre CD151 a été généré chez la souris par immunisations soustractives avec des cellules humaines de carcinome épidermoïde HEp-3 (Testa et al., 1999, Cancer Res. 59, 3812-3820).

L'anticorps 50-6 est capable d'inhiber *in vitro* la migration des cellules humaines de carcinome cervical HeLa transfectées afin de surexprimer CD151 et des cellules HEp-3, et l'angiogénèse dans un modèle de néovascularisation de membrane chorioallantoïque induite par le bFGF (basic fibroblast growth factor). Il inhibe *in vivo* les métastases induites par inoculation de cellules HEp-3 dans 2 modèles d'embryon de poulet (Testa et al., 1999, Cancer Res. 59, 3812-3820). Dans ces modèles, l'activité inhibitrice de l'anticorps 50-6 est déterminée par la mesure de l'activité de la proteine huPA (human urokinase-type plasminogen activator) dans des extraits de poumons. Selon les auteurs, ce dosage constitue le reflet de la présence de cellules humaines dans les poumons. Après dosage, la réduction des métastases (dissémination des cellules HEp-3 dans les poumons des embryons de poulet) induite par l'anticorps 50-6 est estimée, par comparaison à un anticorps contrôle, à 74% dans un modèle dit de « métastase spontanée » dans lequel l'inoculation des cellules est suivie par une injection de l'anticorps, et à 57% dans un modèle dit de « métastase expérimentale » dans lequel les cellules et l'anticorps sont inoculés conjointement. Selon les auteurs, les propriétés anti-tumorales de l'anticorps 50-6 observées *in vivo* ne semblent pas être liées à un effet cytostatique ou cytotoxique puisqu'il n'a montré aucun effet sur la prolifération *in vitro* des cellules HEp-3.

L'hybridome produisant l'anticorps 50-6 est disponible à l'ATCC sous la référence CRL-2696 (hybridome déposé initialement sous la référence 50-6 [PTA-227]).

Selon un aspect général, la présente invention vise l'utilisation d'au moins un anticorps, ou l'un de ses fragments fonctionnels, capable de se lier à la protéine CD151 et ainsi d'inhiber la croissance tumorale pour la préparation d'un médicament destiné au traitement des tumeurs primaires.

Plusieurs études expérimentales ont montré le rôle majeur des tétraspanines dans la formation de métastases, en agissant soit comme suppresseurs, soit comme promoteurs de métastases. Ainsi, la transfection de tétraspanines comme CD9, CD63 ou CD82 réduit le potentiel métastatique de lignées cancéreuses. En revanche, l'expression des tétraspanines CD151 et Co-029 semble produire l'effet inverse. Ces 2 tétraspanines seraient donc des promoteurs de la métastase. Ces résultats sont cohérents avec différentes études cliniques qui ont démontré que, dans plusieurs cancers (sein, poumon, oesophage, estomac, foie, pancréas, côlon, prostate, mélanome...), CD9 et CD82 sont moins exprimés sur les tumeurs primitives lorsqu'il y a métastase et que leur baisse d'expression prédit un taux de survie inférieur. Dans le cancer du poumon, la diminution combinée de l'expression de CD9 et de CD82 a été corrélée à un potentiel métastatique plus important que lorsque l'expression d'un seul de ces deux antigènes est réduite.

Plusieurs études rétrospectives ont montré que la surexpression de CD 151 était associée à l'agressivité de certains cancers, comme les cancers du poumon, du colon et de la prostate, et pouvait être considérée comme un facteur de mauvais pronostic (Tokuhara et al., 2001, Clin. Cancer Res. 7, 4109-4114 ; Hashida et al., 2003, Br. J. Cancer 89, 158-167 ; Ang et al., 2004, Cancer Epidemiol. Biomarkers Prev. 13, 1717-1721). Dans ces cas, la moyenne de survie est en effet diminuée chez les patients dont la tumeur exprime CD151, comparativement à ceux dont la tumeur n'exprime pas CD151.

La surexpression de CD151 dans différentes lignées tumorales humaines (HeLa, RPMI14788, A172, HT1080), induite par transfection du gène correspondant, provoque une augmentation de la motilité, de la migration et de l'invasion des cellules transfectées (Testa et al., 1999, Cancer Res. 59, 3812-3820 ; Kohno et al., 2002, Int. J. Cancer 97, 336-343). Ces phénomènes sont inhibés en présence d'anticorps anti-CD151.

Selon un autre aspect, les fragments fonctionnels d'anticorps selon l'invention consistent, par exemple, en des fragments Fv, scFv (sc pour simple chaîne), Fab, F(ab')₂, Fab', scFv-Fc ou diabodies, ou tout fragment dont la durée de demie-vie aurait été augmentée par modification chimique, comme l'ajout de poly(alkylène) glycol tel que le poly(éthylène)glycol ("PEGylation") (fragments PEGylés appelés Fv-PEG, scFv-PEG, Fab-PEG, F(ab')₂-PEG ou Fab'-PEG) (« PEG » d'après la nomenclature anglaise Poly(Ethylene) Glycol), ou par incorporation dans un liposome, microsphères ou PLGA, lesdits fragments étant capables d'exercer de manière générale une activité même partielle de l'anticorps dont ils sont issus.

De préférence, lesdits fragments fonctionnels seront constitués ou comprendront une séquence partielle de la chaîne variable lourde ou légère de l'anticorps dont ils sont dérivés, ladite séquence partielle étant suffisante pour retenir la même spécificité de liaison que l'anticorps dont elle est issue et une affinité suffisante, de préférence au moins égale à 1/100, de manière plus préférée à au moins 1/10 de celle de l'anticorps dont elle est issue.

Un tel fragment fonctionnel comportera au minimum 5 acides aminés, de préférence 10, 15, 25, 50 et 100 acides aminés consécutifs de la séquence de l'anticorps dont il est issu.

De préférence, ces fragments fonctionnels seront des fragments de type Fv, scFv, Fab, F(ab')₂, F(ab'), scFv-Fc ou diabodies, qui possèdent généralement la même spécificité de fixation que l'anticorps dont ils sont issus. Selon la présente invention, des fragments d'anticorps de l'invention peuvent être obtenus à partir des anticorps tels que décrits précédemment par des méthodes telles que la digestion par des enzymes, comme la pepsine ou la papaïne et/ou par clivage des ponts disulfures par réduction chimique. D'une autre manière les fragments d'anticorps compris dans la présente invention peuvent être obtenus par des techniques de recombinaisons génétiques bien connues également de l'homme de l'art ou encore par synthèse peptidique au moyen par exemple de synthétiseurs automatiques de peptides tels que ceux fournis par la société Applied.

Selon un aspect de l'invention, l'anticorps utilisé consiste en un anticorps monoclonal murin.

Sont également compris par anticorps selon la présente invention, les anticorps chimériques ou humanisés.

Par anticorps chimérique, on entend désigner un anticorps qui contient une région variable (chaîne légère et chaîne lourde) naturelle dérivée d'un anticorps d'une espèce donnée en association avec les régions constantes de chaîne légère et chaîne lourde d'un anticorps d'une espèce hétérologue à ladite espèce donnée.

Les anticorps ou leurs fragments de type chimérique utilisés selon l'invention peuvent être préparés en utilisant les techniques de recombinaison génétique. Par exemple, l'anticorps chimérique pourra être réalisé en clonant un ADN recombinant comportant un promoteur et une séquence codant pour la région variable d'un anticorps monoclonal non humain, notamment murin, selon l'invention et une séquence codant pour la région constante d'anticorps humain. Un anticorps chimérique de l'invention codé par un tel gène recombinant sera par exemple une chimère souris-homme, la spécificité de cet anticorps étant déterminée par la région variable dérivée de l'ADN murin et son isotype déterminé par la région constante dérivée de l'ADN humain. Pour les méthodes de préparation d'anticorps chimériques, on pourra par exemple se référer au document Verhoeyn et al. (BioEssays, 8:74, 1988).

Par anticorps humanisés, on entend désigner un anticorps qui contient des régions CDRs dérivées d'un anticorps d'origine non humaine, les autres parties de la molécule d'anticorps étant dérivée d'un (ou de plusieurs) anticorps humains. En outre, certains des résidus des segments du squelette (dénommés FR) peuvent être modifiés pour conserver l'affinité de liaison (Jones et al., Nature, 321:522-525, 1986 ; Verhoeyen et al., Science, 239:1534-1536, 1988 ; Riechmann et al., Nature, 332:323-327, 1988).

Les anticorps humanisés ou leurs fragments fonctionnels peuvent être préparés par des techniques connues de l'homme de l'art (comme par exemple celles décrites dans les documents Singer et al., J. Immun. 150:2844-2857, 1992 ; Mountain et al., Biotechnol. Genet. Eng. Rev., 10:1-142, 1992 ; ou Bebbington et al., Bio/Technology, 10:169-175, 1992). De tels anticorps humanisés sont préférés pour leur utilisation dans des méthodes de traitement prophylactique et/ou thérapeutique *in vivo.* D'autres techniques d'humanisation sont également connues de l'homme de l'art comme par exemple la technique du « *CDR Grafting »* décrite par PDL faisant l'objet des brevets EP 0 451 261, EP 0 682 040, EP 0 939 127, EP 0 566 647 ou encore US 5,530,101, US 6,180,370, US 5,585,089 et US 5,693,761. On peut également citer les brevets US 5,639,641 ou encore 6,054,297, 5,886,152 et 5,877,293.

De manière surprenante, et contre toute attente de la part de l'homme de l'art, la présente invention décrit pour la première fois l'utilisation d'un anticorps anti-CD151 tel que décrit plus haut, ou l'un de ses fragments fonctionnels, capable d'inhiber la prolifération des cellules tumorales et le développement de la tumeur primaire, indépendamment de sa capacité à inhiber l'angiogénèse et/ou la formation des métastases.

Les anticorps décrits dans la demande présentent donc la capacité à inhiber très en amont le développement des tumeurs.

Cette activité anti-tumorale des anticorps objet de la présente invention constitue une propriété nouvelle et inattendue pour un anticorps dirigé contre CD151, puisque aucun anticorps anti-CD151 décrit à ce jour ne possède ce type d'activité. Les anticorps objet de la présente invention présentent donc une propriété différente, et supplémentaire, par rapport aux anticorps précédemment décrit, et notamment par rapport à l'anticorps 50-6 puisque les propriétés anti-tumorales de cet anticorps ne sont pas liées à un effet sur la prolifération des cellules tumorales.

Ce résultat constitue aussi la première mise en évidence d'un lien entre CD151 et le développement de la tumeur primaire, voire la prolifération des cellules tumorales in vivo. Seules les activités pro-métastatique et pro-angiogénique de CD151 avaient en effet été décrites à ce jour.

La prolifération désordonnée des cellules d'un organe ou d'un tissu constitue l'une des premières étapes du cancer. Les cellules tumorales sont des cellules qui n'ont plus la capacité d'être soumises aux contrôles normaux de la croissance cellulaire au sein de l'organe ou du tissu concerné. La croissance tumorale est exponentielle, et les cellules tumorales se multiplient de façon excessive sous l'effet de facteurs de croissance et angiogéniques.

Selon un aspect principal, l'invention concerne l'utilisation d'au moins un anticorps anti-CD151, ou l'un de ses fragments fonctionnels, capable d'inhiber le développement de la tumeur primaire et la prolifération des cellules tumorales.

Plus particulièrement, la présente invention concerne l'utilisation d'au moins un anticorps, ou l'un de ses fragments fonctionnels, capable de se lier à la protéine CD151, pour la préparation d'un médicament destiné au traitement des tumeurs primaires.

En outre, la demanderesse avance, sans vouloir être liée par une quelconque théorie, que l'utilisation d'anticorps anti-CD151 dans le cadre du traitement du cancer présenterait un intérêt, non pas uniquement du fait d'une inhibition de l'angiogénèse, mais également du fait de l'inhibition de l'activité promotrice de métastase de CD151.

La présente invention décrit donc l'utilisation d'un anticorps tel que décrit plus haut, ou l'un de ses fragments fonctionnels, capable d'inhiber l'activité promotrice de métastase de ladite protéine CD151 au sein des cellules tumorales primaires.

Plus particulièrement, la demanderesse pense que cette inhibition se traduit par une inhibition des différentes étapes du processus métastatique, et notamment de l'adhésion cellulaire, de la migration cellulaire et/ou de l'invasion cellulaire.

Les étapes classiques de ladite activité promotrice, et plus particulièrement de la dissémination tumorale et du processus métastatique, sont les suivantes :
1/ l'invasion du tissu de soutien par les cellules de la tumeur primaire qui nécessite une dégradation par des enzymes protéolytiques (telles que les métalloprotéinases) de la membrane basale et de la matrice extracellulaire constituées de protéines structurales comme la laminine, le collagène ou la fibronectine,
2/ la migration des cellules tumorales à travers les tissus et dans la circulation sanguine,
3/ l'adhésion à la paroi vasculaire et l'arrêt dans un organe,
4/ la sortie du vaisseau (nouvelle étape d'invasion) et l'adaptation au nouvel environnement (prolifération et angiogénèse).

La migration cellulaire est essentielle pendant le développement embryonnaire. Bien que la migration des cellules soit moins importante chez l'adulte, certains types cellulaires, tels que les lymphocytes, les macrophages et les fibroblastes, continueront à se déplacer durant la réponse immunitaire, l'inflammation et la guérison de plaie chez l'adulte pour maintenir l'homéostasie. Cependant, au niveau pathologique, la migration des cellules tumorales, contribue de façon majeure à la progression de tumeurs au stade métastatique. Un certain nombre de facteurs chimiotactiques sont responsables de cette migration, facteurs dérivés soit des cellules tumorales, soit de l'hôte. Parmi ces facteurs, on cite les facteurs de croissance (notamment ceux qui stimulent l'angiogénèse), les peptides de dégradation du collagène, les protéines d'adhésion comme la laminine et la fibronectine.

Selon un aspect particulier, la demande concerne l'utilisation d'au moins un anticorps anti-CD151, ou l'un de ses fragments fonctionnels, capable d'inhiber la migration cellulaire des cellules tumorales.

L'invasion est le principal signe de la malignité d'une tumeur : celle-ci déborde son siège d'origine pour s'étendre dans les tissus voisins et à distance. Le caractère invasif traduit la perte des propriétés habituelles d'une cellule : normalement les cellules de la plupart des tissus adhèrent les unes aux autres par des structures appelées desmosomes, par des molécules adhésives; dans un épithélium, elles adhèrent aussi à la membrane basale qui le limite en profondeur. Les cellules tumorales perdent ces propriétés normales pour en acquérir de nouvelles. Les liens entre elles se relâchent et les cellules se libèrent les unes des autres. Elles acquièrent une mobilité qui leur permet de se détacher du foyer primitif, de s'insinuer dans (d'envahir) les tissus voisins, en suivant parfois les fibres du tissu conjonctif. Pour les épithéliums normalement bordés par une membrane basale et pour les carcinomes qui en dérivent, cette membrane est le premier obstacle à franchir. Elle est altérée et dissoute par des enzymes (protéases, cathepsine) sécrétées par les cellules tumorales. Cette destruction de la membrane basale est parfois accentuée par des enzymes normalement sécrétées par des globules blancs et détournées de leurs activités habituelles. Toutes ces modifications biologiques et moléculaires du comportement cellulaire sont la condition de l'invasion.

Selon un autre aspect, la demande concerne l'utilisation d'au moins un anticorps anti-CD151, ou l'un de ses fragments fonctionnels, capable d'inhiber l'invasion cellulaire des cellules tumorales.

Les cellules de l'organisme adhèrent les unes aux autres ainsi qu'à la matrice extracellulaire qui les entoure. L'adhésion cellulaire est un mécanisme ubiquitaire impliqué dans la plupart des phénomènes cellulaires physiologiques, tels que la survie, la prolifération ou la différentiation des cellules, mais aussi dans différentes situations pathologiques comme par exemple le cancer et le phénomène de métastase. Différentes protéines de la surface cellulaire sont impliquées dans l'adhésion cellulaire, telles que les cadhérines ou les intégrines.

De manière préférée, l'utilisation selon l'invention repose principalement sur l'inhibition de l'adhésion cellulaire.

Selon encore un autre aspect, l'invention concerne l'utilisation d'au moins un anticorps anti-CD151, ou l'un de ses fragments fonctionnels, capable d'inhiber l'adhésion cellulaire des cellules tumorales.

Comme cela a été dit plus haut, la protéine CD151 fait partie de la famille des tétraspanines et, à ce titre, comprend 2 domaines extracellulaires EC1 (18 acides aminés, séquence [40-57]) et EC2 (109 acides aminés, séquence [113-221]), appelés aussi boucles extracellulaires.

Selon la présente invention, les anticorps utilisés sont capables de se lier à au moins un épitope situé dans le domaine extracellulaire. De manière préférée, ledit anticorps se fixera au niveau des boucles EC 1 et/ou EC2.

Plus particulièrement, selon une forme d'exécution préférée de l'invention, il est décrit l'utilisation d'au moins un anticorps anti-CD151, ou l'un de ses fragments fonctionnels, capable de se lier à un épitope compris dans la boucle extra-cellulaire 1 (EC1) et/ou 2 (EC2), préférentiellement EC2, correspondant respectivement aux acides-aminés 40-57 (SEQ ID No. 6) et 113-221 (SEQ ID No.4) de la protéine CD151.

La boucle EC1 [40-57] comprend 18 acides-aminés et présente une masse théorique de 2002,2 Da.

La boucle EC2 [113-221] possède un site de N-glycosylation (résidu Asn159) et 6 résidus Cystéine formant 3 ponts disulfure. Un modèle structural de la boucle EC2 des tétraspanines, et notamment de CD151, a été proposé à partir de la structure tridimensionnelle de la boucle EC2 de la tétraspanine CD81 (Seigneuret et al., 2001, J. Biol. Chem. 276, 40055-40064). Selon ce modèle, les tétraspanines possèdent une charpente commune, relativement conservée et constituée de 3 hélices α, et un domaine spécifique variable. Pour CD151, cette charpente serait constituée des régions [113-157] et [209-221], et le domaine variable de la région [158-208].

Le domaine variable de la boucle EC2 serait plus particulièrement impliqué dans les interactions spécifiques de CD151 avec des protéines de la famille des intégrines. Des expériences de mutation dirigée ont notamment montré l'importance de la région [193-208], et plus précisément du tripeptide QRD [194-196] et du résidu Cystéine en position 192, dans l'association de CD151 avec certaines intégrines récepteurs de la laminine, telles que les intégrines α3β1 ou α6β4 (Kazarov et al., 2002, J. Cell Biol. 158, 1299-1309).

De manière encore plus préférée, la présente invention vise l'utilisation d'au moins un anticorps anti-CD151, ou l'un de ses fragments fonctionnels, capable de se lier à un épitope de la région EC2 comprenant au moins les acides-aminés Glutamine, Arginine et Aspartate respectivement aux positions 194, 195 et 196 (QRD¹⁹⁴⁻¹⁹⁶) de la protéine CD151

Selon un autre aspect, il faut comprendre que l'invention consiste principalement en l'utilisation d'au moins un anticorps anti-CD151, ou l'un de ses fragments fonctionnels, qui consiste en un anticorps monoclonal.

Il faut entendre par « anticorps monoclonal » un anticorps issu d'une population d'anticorps sensiblement homogènes. Plus particulièrement, les anticorps individuels d'une population sont identiques à l'exception de quelques mutations éventuelles pouvant se produire naturellement qui peuvent se retrouver en proportions minimes. En d'autres termes, un anticorps monoclonal consiste en un anticorps homogène résultant de la prolifération d'un seule clone cellulaire (par exemple un hybridome, une cellule hôte eucaryote transféctée avec une molécule d'ADN codant pour l'anticorps homogène, une cellule hôte procaryote transféctée avec une molécule d'ADN codant pour l'anticorps homomgène, etc.) et qui est généralement caractérisé par des chaînes lourdes d'une seule et même classe et sous-classe, et des chaînes légères d'un seul type. Les anticorps monoclonaux sont fortement spécifiques et sont dirigés contre un seul antigène. En outre, contrairement aux préparations d'anticorps polyclonaux qui comprennent classiquement différents anticorps dirigés contre différents déterminants, ou épitopes, chaque anticorps monoclonal est dirigé contre un seul épitope de l'antigène.

Selon un mode de réalisation particulier de l'invention, l'anticorps monoclonal utilisé est choisis parmi les anticorps TS151 et TS151r. Dans la suite de la description, les expressions TS151r et TS151R sont interchangeables.

La présente invention décrit donc l'utilisation d'au moins un anticorps anti-CD151, ou l'un de ses fragments fonctionnels, ledit anticorps consistant en l'anticorps TS151 et/ou l'anticorps TS151r.

Plus particulièrement, l'anticorps TS151 est défini en ce qu'il comprend au moins :
- les 3 CDRs de la chaîne lourde CDR-H1, CDR-H2 et CDR-H3 respectivement de séquence SEQ ID No. 7, 8 et 9 ; et
- les 3 CDRs de la chaîne légère CDR-L1, CDR-L2 et CDR-L3 respectivement de séquence SEQ ID No. 11, 12 et 13.

Selon une autre forme de réalisation, l'anticorps TS151 est caractérisé en ce qu'il comprend une chaîne lourde comprenant la séquence SEQ ID No. 10 et une chaîne légère comprenant la séquence SEQ ID No. 14.

Le tableau 1 ci-dessous reprend ces éléments.

**Tableau 1**

| **Anticorps** | **Chaîne légère** | **Chaîne lourde** | **SEQ ID N°** |
|---|---|---|---|
| TS151 | | CDR-H1 | 7 |
| | | CDR-H2 | 8 |
| | | CDR-H3 | 9 |
| | CDR-L1 | | 11 |
| | CDR-L2 | | 12 |
| | CDR-L3 | | 13 |
| | | Entière (domaine variable) | 10 |
| | Entière (domaine variable) | | 14 |

Pour ce qui est de l'anticorps TS151r, ce dernier est défini en ce qu'il comprend au moins :
- les 3 CDRs de la chaîne lourde CDR-H1, CDR-H2 et CDR-H3 respectivement de séquence SEQ ID No. 15, 16 et 17 ; et
- les 3 CDRs de la chaîne légère CDR-L1, CDR-L2 et CDR-L3 respectivement de séquence SEQ ID No. 19, 20 et 21.

Selon une autre forme de réalisation, l'anticorps TS151r est caractérisé en ce qu'il comprend une chaîne lourde comprenant la séquence SEQ ID No. 18 et une chaîne légère comprenant la séquence SEQ ID No. 22.

Le tableau 2 ci-dessous résume ces éléments.

**Tableau 2**

| **Anticorps** | **Chaîne légère** | **Chaîne lourde** | **SEQ ID N^{o}** |
|---|---|---|---|
| TS151r | | CDR-H1 | 15 |
| | | CDR-H2 | 16 |
| | | CDR-H3 | 17 |
| | CDR-L1 | | 19 |
| | CDR-L2 | | 20 |
| | CDR-L3 | | 21 |
| | | Entière (domaine variable) | 18 |
| | Entière (domaine variable) | | 22 |

Selon une autre forme de réalisation, le tableau 3 ci-dessous regroupe les séquences nucléotidiques des anticorps TS151 et TS151r.

**Tableau 3**

| **Anticorps** | **Chaîne légère** | **Chaîne lourde** | **SEQ ID N°** |
|---|---|---|---|
| TS151 | | CDR-H1 | 23 |
| | | CDR-H2 | 24 |
| | | CDR-H3 | 25 |
| | CDR-L1 | | 27 |
| | CDR-L2 | | 28 |
| | CDR-L3 | | 29 |
| | | Entière (domaine variable) | 26 |
| | Entière (domaine variable) | | 30 |
| TS151r | | CDR-H1 | 31 |
| | | CDR-H2 | 32 |
| | | CDR-H3 | 33 |
| | CDR-L1 | | 35 |
| | CDR-L2 | | 36 |
| | CDR-L3 | | 37 |
| | | Entière (domaine variable) | 34 |
| | Entière (domaine variable) | | 38 |

La génération desdits anticorps est détaillée au niveau de l'exemple 1. Ces 2 anticorps sont dirigés contre des épitopes différents puisque le TS151 reconnaît CD151 aussi bien lorsqu'il est associé aux intégrines ou libre à la surface de la cellule (Chometon et al., 2006, Exp. Cell Res. 312, 983-995), alors que le TS151r ne reconnaît pas les complexes CD151-intégrines (Serru et al., 1999, Biochem. J. 340, 103-111 ; Geary et al., 2001, Tissue Antigens 58, 141-153 ; Kazarov et al., 2002, J. Cell Biol. 158, 1299-1309 ; Sterk et al., 2002, J. Cell Sci. 115, 1161-1173). L'épitope reconnu par le TS151r est localisé dans la boucle EC2 et comporte les résidus Q194, R195 et D196 (Kazarov et al., 2002, J. Cell Biol. 158, 1299-1309). Cet anticorps est donc, en partie au moins, dirigé contre le site de CD151 impliqué dans les interactions avec les intégrines. Le résidu C192 serait lui aussi impliqué dans la reconnaissance de CD151 par le TS151r (Kazarov et al., 2002, J. Cell Biol. 158, 1299-1309). L'épitope de l'anticorps TS151, bien qu'il soit différent de celui du TS151r, n'a pas été déterminé avec précision.

Le traitement de kératinocytes humains (lignée épithéliale HaCaT) par l'anticorps TS151r provoque une perte du contact cellule-cellule, un réarrangement du cytosquelette, une redistribution intracellulaire de l'intégrine α6β4 et une augmentation de la migration des cellules sur la laminine 1 (Chometon et al., 2006, Exp. Cell Res. 312, 983-995).

Plus particulièrement, l'anticorps utilisé préféré consiste en l'anticorps TS151.

De manière préférée, l'utilisation des anticorps anti-CD151 dans le cadre du traitement du cancer se justifie tout particulièrement dans les cancers surexprimant ce même récepteur CD 151.

De tels cancers consistent en le cancer du colon [Hashida et al., Br. J. Cancer 89 (2003) :158-167], le cancer du poumon, préférentiellement du poumon non à petites cellules [Tokuhara et al., Clin. Cancer Res. 7 (2001) :4109-4114], le cancer de la prostate [Ang et al., Cancer Epidemiol. Biomarkers 13 (2004) :17] et le cancer du pancréas [Gesierich et al., Clin. Cancer Res. 11 [2005) :2840-2852].

La présente demande revendique donc l'utilisation d'un anticorps tel que décrit plus haut pour le traitement du cancer, ledit cancer consistant préférentiellement en les cancers du colon, du poumon, de la prostate ou du pancréas.

L'invention concerne également une composition pharmaceutique comprenant à titre de principe actif un composé consistant en un anticorps, ou l'un de ses composés dérivés ou fragments fonctionnels, de préférence additionné d'un excipient et/ou d'un véhicule pharmaceutiquement acceptable.

Plus particulièrement, l'invention vise l'utilisation d'un anticorps selon l'invention pour la préparation d'une composition pharmaceutique comprenant, en outre, au moins un véhicule pharmaceutiquement acceptable

Dans la présente description, on entend désigner par véhicule pharmaceutiquement acceptable, un composé ou une combinaison de composés entrant dans une composition pharmaceutique ne provoquant pas de réactions secondaires et qui permet par exemple la facilitation de l'administration du ou des composés actifs, l'augmentation de sa durée de vie et/ou de son efficacité dans l'organisme, l'augmentation de sa solubilité en solution ou encore l'amélioration de sa conservation. Ces véhicules pharmaceutiquement acceptables sont bien connus et seront adaptés par l'homme de l'art en fonction de la nature et du mode d'administration du ou des composés actifs choisis.

De préférence, ces composés seront administrés par voie systémique, en particulier par voie intraveineuse, par voie intramusculaire, intradermique, intrapéritonéale ou sous-cutanée, ou par voie orale. De manière plus préférée, la composition comprenant les anticorps selon l'invention, sera administrée à plusieurs reprises, de manière étalée dans le temps.

Leurs modes d'administration, posologies et formes galéniques optimaux peuvent être déterminés selon les critères généralement pris en compte dans l'établissement d'un traitement adapté à un patient comme par exemple l'âge ou le poids corporel du patient, la gravité de son état général, la tolérance au traitement et les effets secondaires constatés.

Selon la demande, il est décrit une composition pour le traitement du cancer, caractérisée en ce qu'elle comprend à titre de principe actif au moins un anticorps anti-CD151, ou l'un de ses fragments fonctionnels, capable de se lier à la protéine CD151.

Selon la demande, il est décrit une composition pour le traitement du cancer, caractérisée en ce qu'elle comprend à titre de principe actif au moins un anticorps anti-CD151, ou l'un de ses fragments fonctionnels, capable de se lier à la protéine CD151 et/ou d'inhiber son activité promotrice de métastase.

Selon la demande, il est également décrit une composition pour le traitement du cancer, caractérisée en ce qu'elle comprend à titre de principe actif au moins un anticorps anti-CD151, ou l'un de ses fragments fonctionnels, capable d'inhiber le développement de tumeurs primaires.

Selon un autre aspect e l'invention, il est décrit une composition comprenant au moins un anticorps anti-CD151, ou l'un de ses fragments fonctionnels, ledit au moins un anticorps étant un anticorps monoclonal sélectionné parmi les anticorps TS151 ou TS151r.

Selon encore un aspect de l'invention, il est revendiquée une composition qui comprend une combinaison des anticorps TS151 et TS151r, ou de leur fragments fonctionnels.

Il ressort de la littérature que la protéine CD151 est surexprimée dans les cancers et, tout particulièrement, dans les carcinomes du colon [Hashida et al., Br. J. Cancer 89 (2003): 158-167], les cancers du poumon non à petites cellules [Tokuhara et al., Clin. Cancer Res. 7 (2001): 4109-4114], les cancers de la prostate [Ang et al., Cancer Epidemiol. Biomarkers 13 (2004): 1717-1721] et les cancers du pancréas [Gesierich et al., Clin. Cancer Res. 11 (2005): 2840-2852].

Bien évidemment, la liste ci-dessus n'est donnée qu'à titre illustratif et tout cancer doit être compris comme surexprimant la protéine CD151 et donc susceptible d'être traité conformément à la présente invention.

Une autre forme d'exécution complémentaire de l'invention consiste en une composition telle que décrite plus haut qui comprend, en outre, comme produit de combinaison pour une utilisation simultanée, séparée ou étalée dans le temps, un agent cytotoxique/cytostatique et/ou un anticorps monoclonal.

La présente invention concerne donc également une composition telle que décrite plus haut, caractérisée en ce qu'elle comprend, en outre, comme produit de combinaison pour une utilisation simultanée, séparée ou étalée dans le temps, au moins un agent cytotoxique/cytostatique et/ou une toxine cellulaire et/ou un radioélément et/ou un anticorps monoclonal.

On entend par "utilisation simultanée", l'administration des deux composés de la composition selon l'invention compris dans une seule et même forme pharmaceutique.

On entend par "utilisation séparée", l'administration, en même temps, des deux composés de la composition selon l'invention, compris dans des formes pharmaceutiques distinctes.

On entend par "utilisation étalée dans le temps", l'administration successive des deux composés de la composition selon l'invention, compris chacun dans une forme pharmaceutique distincte.

D'une façon générale, la composition selon l'invention augmente considérablement l'efficacité du traitement du cancer. En d'autres termes, l'effet thérapeutique de l'anticorps selon l'invention est potentialisé de manière inattendue par l'administration d'un agent cytotoxique. Un autre avantage subséquent majeur produit par une composition selon l'invention, concerne la possibilité d'utiliser des doses efficaces en principe actif plus faibles, ce qui permet d'éviter ou de réduire les risques d'apparition des effets secondaires, en particulier l'effet de l'agent cytotoxique. De plus, cette composition selon l'invention permettrait d'atteindre l'effet thérapeutique escompté plus rapidement.

Par « agents thérapeutiques anti-cancer » ou « agents cytotoxiques », il faut comprendre une substance qui, lorsqu'elle est administrée à un patient, traite ou prévient le développement du cancer chez le patient. A titre d'exemple non limitatif pour de tels agents, il peut être mentionné les agents « alkylant », les antimétabolites, les antibiotiques anti-tumoraux, les inhibiteurs mitotiques, les inhibiteurs de fonction chromatine, les agents anti-angiogénèse, les anti-oestrogène, les anti-androgène ou les immuno-modulateurs.

De tels agents sont, par exemple, cités dans le VIDAL, à la page consacrée aux composés attachés à la cancérologie et l'hématologie colonne « Cytotoxiques », ces composés cytotoxiques cités par référence à ce document sont cités ici comme agents cytotoxiques préférés.

Les « agents alkylants » font référence à toute substance qui peut se coupler de manière covalente ou alkyler toute molécule, préférentiellement un acide nucléique (ex. : ADN), au sein d'une cellule. Comme exemples de tels agents alkylants, il peut être cité les moutardes à l'azote comme la méchloréthamine, le chlorambucil, le melphalan, le chlorhydrate, le pipobroman, la prednimustine, le phosphate disodique ou l'estramustine ; les oxazaphosphorines comme la cyclophosphamide, l'altretamine, la trofosfamide, la sulfofosfamide ou l'ifosfamide ; les aziridines ou ethylènes-imines comme le thiotepa, la triéthylèneamine ou l'altétramine ; les nitrosourées comme la carmustine, la streptozocine, la fotémustine ou la lomustine ; les sulfonates d'alkyle comme le busulfan, le tréosulfan ou l'improsulfan ; les triazènes comme la dacarbazine ; ou encore les complexes du platine comme le cisplatine, l'oxaliplatine ou le carboplatine.

Les « antimétabolites » font référence à des substances qui bloquent la croissance et/ou le métabolisme cellulaire en interférant avec certaines activités, généralement la synthèse d'ADN. A titre d'exemple d'antimétabolite, il peut être mentionné les methotrexate, 5-fluorouracile, floxuridine, 5-fluorodeoxyuridine, capecitabine, cytarabine, fludarabine, cytosine arabinoside, 6-mercaptopurine (6-MP), 6-thioguanine (6-TG), chlorodésoxyadénosine, 5-azacytidine, gemcitabine, cladribine, deoxycoformycine et la pentostatine.

Les « antibiotiques anti-tumoraux » font référence aux composés qui peuvent prévenir ou inhiber la synthèse d'ADN, d'ARN et/ou de protéines. Des exemples de tels antibiotiques anti-tumoraux comprennent la doxorubicine, daunorubicine, idarubicine valrubicine, mitoxantrone, dactinomycine, mithramycine, plicamycine, mitomycine C, bleomycine, et la procarbazine.

Les « inhibiteurs mitotiques » préviennent la progression normale du cycle cellulaire et de la mitose. En general, les inhibiteurs des microtubules ou « taxoides » comme le paclitaxel et le docétaxel sont capables d'inhiber la mitose. Les alcaloîdes de vinca, comme la vinblsatine, la vincristine, la vindésine et la vinorelbine sont également capables d'inhiber la mitose.

Les « inhibiteurs de fonction chromatine » ou « inhibiteurs de topo-isomérases » font référence à des substances qui inhibent la fonction normale des protéines modélant la chromatine comme les topo-isomérases I et II. Des exemples de tels inhibiteurs comprennent, pour la topo-isomérase I, la camptothécine ainsi que ses dérivés comme l'irinotécan ou le topotécan et, pour la topo-isomérase II, l'étoposide, le phosphate d'étiposide et le téniposide.

Les « agents anti-angiogénèse » font références à toute drogue, composé, substance ou agent qui inhibe la croissance des vaisseaux sanguins. Des exemples d'agents anti-angiogénèse comprennent, sans aucune limitation, les razoxin, marimastat, batimastat, prinomastat, tanomastat, ilomastat, CGS-27023A, halofuginone, COL-3, neovastat, BMS-275291, thalidomide, CDC 501, DMXAA, L-651582, squalamine, endostatine, SU5416, SU6668, interferon-alpha, EMD121974, interleukine-12, IM862, angiostatine et la vitaxine.

Les « anti-oestrogène » ou « agents anti-oestrogénique » font référence à toute substance qui diminue, antagonise ou inhibe l'action des oestrogènes. Des exemples de tels agents sont les tamoxifène, toremifène, raloxifène, droloxifène, iodoxyfène, anastrozole, letrozole, et l'exemestane.

Les « anti-androgènes » ou « agents anti-androgène » font référence à toute substance qui réduit, antagonise ou inhibe l'action d'un androgène. Des exemples d'anti-androgènes sont les flutamide, nilutamide, bicalutamide, sprironolactone, cyproterone acetate, finasteride et la cimitidine.

Les immunomodulateurs sont des substances qui stimulent le système immunitaire. Des exemples de tels immunomodulateurs comprennent les interférons, les interleukines comme l'aldesleukine, OCT-43, denileukin diflitox ou l'interleukine-2, les facteurs de nécrose tumorale comme la tasonermine, ou d'autres types d'immunomodulateurs comme le lentinan, le sizofiran, le roquinimex, le pidotimod, la pégadémase, la thymopentine, le poly I :C, ou le levamisole en combinaison avec le 5-fluorouracil.

Pour plus de détails, l'homme de l'art pourra se reporter au manuel édité par l'Association Française des Enseignants de Chimie Thérapeutique intitulé « traité de chimie thérapeutique, Vol. 6, Médicaments antitumoraux et perspectives dans le traitement des cancers, édition TEC & DOC, 2003 ».

Les anticorps monoclonaux préférés sont choisis parmi les anticorps capables d'inhiber spécifiquement l'activité tyrosine kinase des récepteurs IGF-IR, EGFR, HER2/neu, cMET, VEGFR, VEGF, etc., (ou tout autre anticorps anti-tumoral connu de l'homme de l'art), isolés, ou leurs fragments fonctionnels et composés dérivés, capables d'inhiber l'activité proliférative et/ou anti-apoptotique et/ou angiogénique et/ou inductrice de dissémination métastatique promues par lesdits récepteurs.

Dans un mode de réalisation particulièrement préféré, ladite composition comme produit de combinaison selon l'invention est caractérisée en ce que ledit agent cytotoxique est couplé chimiquement audit anticorps pour une utilisation simultanée.

Dans un mode de réalisation particulièrement préféré, ladite composition selon l'invention est caractérisée en ce que ledit agent cytotoxique/cytostatique est choisi parmi les agents inhibiteurs ou stabilisateurs du fuseau, de préférence la vinorelbine et/ou la vinflunine et/ou la vincristine.

Afin de faciliter le couplage entre ledit agent cytotoxique et ledit anticorps selon l'invention, on pourra notamment introduire des molécules espaceurs entre les deux composés à coupler, telles que des poly(alkylènes)glycols comme le polyéthylèneglycol, ou encore des acides aminés, ou, dans un autre mode de réalisation, utiliser des dérivés actifs desdits agents cytotoxiques dans lesquels auront été introduites des fonctions capables de réagir avec ledit anticorps selon l'invention. Ces techniques de couplage sont bien connues de l'homme de l'art et ne seront pas développées dans la présente description.

L'invention est, sous un autre aspect, relative à une composition caractérisée en ce que l'un, au moins, desdits anticorps, ou l'un de leur composé dérivé ou fragment fonctionnel, est conjugué avec une toxine cellulaire et/ou un radioélément

De préférence, ladite toxine ou ledit radioélément est capable d'empêcher la croissance ou la prolifération de la cellule tumorale, notamment d'inactiver totalement ladite cellule tumorale.

De préférence encore, ladite toxine est une toxine d'entérobactéries, notamment l'exotoxine A de Pseudomonas.

Les radioéléments (ou radio-isotopes) préférentiellement conjugués à l'anticorps employés en thérapie sont des radio-isotopes qui émettent des rayons gamma et préférentiellement l'iodinel¹³¹, l'yttrium⁹⁰, l'or¹⁹⁹, le palladium¹⁰⁰, le cuivre⁶⁷, le bismuth²¹⁷ et l'antimony²¹¹. Les radio-isotopes qui émettent des rayons beta et alpha peuvent également être utilisés en thérapie.

Par toxine ou radioélément conjugué à au moins un anticorps, ou l'un de leur fragment fonctionnel, selon l'invention, on entend désigner tout moyen permettant de lier ladite toxine ou ledit radioélément audit au moins un anticorps, notamment par couplage covalent entre les deux composés, avec ou sans introduction de molécule de liaison.

Parmi les agents permettant une liaison chimique (covalente), électrostatique ou non covalente de tout ou partie des éléments du conjugué, il peut être mentionné tout particulièrement le benzoquinone, le carbodiimide et plus particulièrement l'EDC (1-ethyl-3-[3-dimethylaminopropyl]-carbodiimide hydrochloride), le dimaleimide, l'acide dithiobis-nitrobenzoic (DTNB), le N-succinimidyl S-acetyl thio-acetate (SATA),les agents dits de « bridging » présentant un ou plusieurs groupements, ayant un ou plusieuyrs groupements phenylaside, réagissant avec les ultraviolets (U.V.) et tout préférentiellement le N-[-4-(azidosalicylamino)butyl]-3'-(2'-pyridyldithio)propionamide (APDP), le N-succinimid-yl 3-(2-pyridyldithio)propionate (SPDP) et le 6-hydrazino-nicotinamide (HYNIC).

Une autre forme de couplage, tout spécialement pour les radioéléments, peut consister en l'utilisation d'un chélateur d'ions bifonctionnel.

Parmi ces chélateurs, il est possible de mentionner les chélates dérivés de l'EDTA (ethylenediaminetetraacetic acid) ou du DTPA (diethylenetriaminepentaacetic acid) qui ont été développés pour lier des métaux, particulièrement des métaux radioactifs, et des immunoglobulines. Ainsi, le DTPA et ses dérivés peut être substitué par différents groupements sur le chaîne de carbones de manière à augmenter la stabilité et la rigidité du complexe ligand-métal (Krejcarek et al. (1977); Brechbiel et al. (1991); Gansow (1991); US patent 4831 175).

Par exemple, le DTPA (diethylenetriaminepentaacetic acid) et ses dérivés, qui a été très largement utilisé en médecine et en biologie pendant longtemps soit sous sa forme libre, soit sous la forme d'un complexe avec un ion métallique, présente la caractéristique remarquable de former des chélates stables avec des ions métalliques et d'être couplé à des protéines d'intérêt thérapeutique ou diagnostique comme des anticorps pour le développement de radio-immunoconjugués en thérapie du cancer (Meases et al., (1984); Gansow et al. (1990)).

La présente invention comprend en outre l'utilisation de la composition selon l'invention pour la préparation d'un médicament.

La présente demande vise donc plus particulièrement l'utilisation d'une composition telle que décrite plus haut pour la préparation d'un médicament destiné au traitement du cancer. Parmi les cancers qui peuvent être prévenus et/ou traités, on préfère le cancer du colon, du poumon, de la prostate ou du pancréas.

En outre, selon un aspect particulièrement innovant et avantageux, la présente invention vise l'utilisation d'une composition telle que décrite plus haut pour la préparation d'un médicament destiné au traitement des tumeurs primaires.

L'invention a également pour objet l'utilisation d'un anticorps selon l'invention, pour la préparation d'un médicament destiné au ciblage spécifique d'un composé biologiquement actif vers des cellules exprimant ou surexprimant le récepteur CD 151.

On entend désigner ici par composé biologiquement actif tout composé capable de moduler, notamment d'inhiber, l'activité cellulaire, en particulier leur croissance, leur prolifération, la transcription ou la traduction de gène.

D'autres caractéristiques et avantages de l'invention apparaissent dans la suite de la description avec les exemples et les figures dont les légendes sont représentées ci-après.

### LEGENDES DES FIGURES

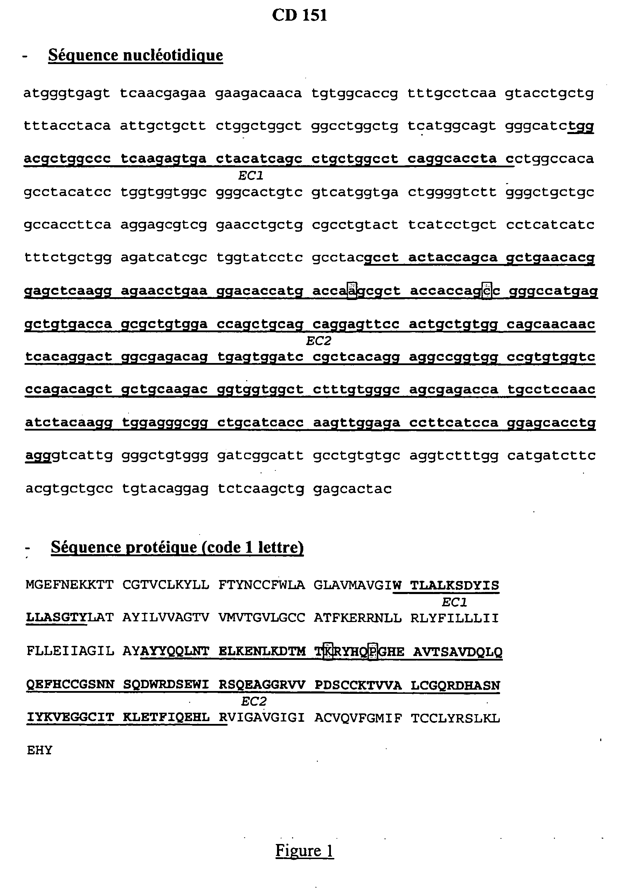

La figure 1 montre les séquences nucléotidiques et protéiques de la protéine CD151, séquences sur lesquelles sont mises en évidences les boucles EC1 et EC2.

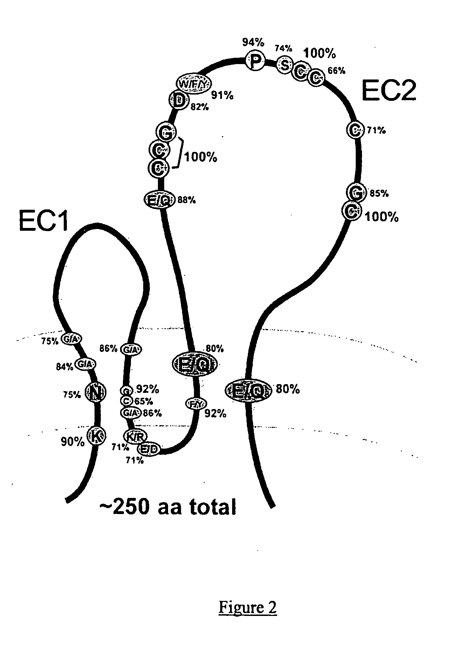

La figure 2 est un schéma illustrant la structure des tétraspanines dont fait parti la protéine CD151, et tout particulièrement les deux boucles extra-cellulaires EC1 et EC2.
La figure 3 représente la comparaison PBS / anticorps contrôle dans le modèle orthotopique A549.
La figure 4 représente l'évaluation de l'activité antitumorale de l'anticorps TS151 *in vivo* dans le modèle orthotopique .Des souris immunodéprimées (n=10) sont greffées par voie intrapleurale avec 1.10⁶ cellules A549. Sept jours après la greffe, les souris sont traitées par voie intrapéritonéale avec une dose d'appel de 500 µg d'anticorps TS151 suivie d'un traitement, deux fois par semaine, durant 5 semaines, avec une dose de 250 µg d'anticorps par souris. Le lot témoin est injecté avec du PBS selon le même schéma d'administration.
La figure 5 illustre l'expression de la molécule CD151 chez des patients atteints de cancer de la prostate. Chaque lettre correspond à l'étude d'un patient et pour chaque patient le panel supérieur correspond au tissus normal adjacent à la tumeur et le panel inférieur correspond au tissus tumoral.
La figure 6 illustre l'expression de la molécule CD151 chez des patients atteints de cancer du poumon. Chaque lettre correspond à l'étude d'un patient et pour chaque patient le panel supérieur correspond au tissus normal adjacent à la tumeur et le panel inférieur correspond au tissus tumoral.
La figure 7 illustre l'activité *in vivo* des anticorps TS151 et TS151R dans le modèle de xénogreffe PC3. Des souris Swiss Nude (n=6) ont été greffées en sous cutanée avec les cellules PC3. Cing jours après greffe des cellules les souris reçoivent, par voie i.p. une dose d'appel de 2 mg/souris des anticorps à tester suivie de deux administrations par semaine d'une dose de 1 mg/souris de ces anticorps. Le volume tumoral est évalué par la formule π/6 x Longueur x largeur x épaisseur et un test de Mann et Whitney est effectué pour l'évaluation statistique des résultats.
La figure 8 illustre l'évaluation de la spécificité des anticorps TS 151 et TS151r pour la forme humaine de CD151 par western blot.
La figure 9 illustre l'inhibition de l'adhésion des cellules A549 sur la laminine 5. A/ Inhibition de l'adhésion cellulaire par différents anticorps anti-intégrines. B/Inhibition de l'adhésion cellulaire par une combinaison TS151/anticorps anti-intégrine α3.

### Exemple 1 : Génération des anticorps TS151r et TS151

### Génération de l'anticorps TS151r

Pour générer l'anticorps TS151r, des souris BALB/c ont été immunisées par voie intrapéritonéale à l'aide de 10⁷ cellules HeLa. Après 3 immunisations et une injection de rappel finale, les cellules de la rate d'une souris ont été fusionnées à des cellules de myélome P3X63AG8 par les techniques classiquement décrites par Kohler et Milstein (5.10⁷ cellules de rate / 3.10⁷ cellules de myélome). Les surnageants des hybridomes résultant de la fusion ont ensuite été criblés sur leur capacité de reconnaissance des cellules HeLa par cytométrie de flux, puis sur leur capacité à immunoprécipiter CD 151 à partir d'un lysat de cellules HeLa en préparé en présence du détergent Brij 97, et à entraîner la co-immunoprécipitation de CD9. L'anticorps TS151r s'est avéré posséder ces différentes propriétés.

### Génération de l'anticorps TS151

Pour générer l'anticorps TS151, des souris BALB/c ont été immunisées par voie intrapéritonéale à l'aide de 10⁷ cellules Jurkat puis 10⁷ HEL (2 immunisations). Après une injection de rappel finale à l'aide de complexes protéiques contenant la protéine ADAM10 obtenus à partir de lysats de cellules Jurkat et HEL, les cellules de la rate ont été fusionnées à des cellules de myélome P3X63AG8 par les techniques classiquement décrites par Kohler et Milstein (5.10⁷ cellules de rate / 3.10⁷ cellules de myélome). Les surnageants des hybridomes résultant de la fusion ont d'abord été criblés sur leur capacité de reconnaissance des cellules Jurkat et HEL par cytométrie de flux. L'anticorps TS 151 a ensuite été sélectionné sur sa capacité à immunoprécipiter CD 151 à partir d'un lysat cellulaire préparé en présence du détergent Brij 97, et à entraîner la co-immunoprécipitation d'autres tétraspanines.

### Exemple 2: Evaluation in vivo de l'activité anti-tumorale des anticorps TS151 et TS151r dans un modèle orthotopique A549

### Matériel et Méthode

Après vérification de l'expression de la protéine CD151 (données non montrées), les cellules A549 originaires de l'ATCC sont cultivées en routine en milieu F12K, 10 mM de glutamine, 10% de SVF. Ces cellules sont divisées 2 jours avant la greffe afin qu'elles soient en phase exponentielle de croissance. Pour la greffe, des souris immunodéprimées de 7 semaines sont anesthésiés avant de recevoir 1.10⁶ cellules A549 par voie intrapleurale. La tumeur primaire se développe rapidement et envahie en 4 jours les structures adjacentes au site d'injection incluant le médiastin, les poumons et le diaphragme. Pour mieux mimer la maladie, le début du traitement ne commence que 7 jours après implantation des cellules, par voie intra péritonéale. Après injection d'une dose d'appel de 500 µg/souris, l'anticorps TS151 purifié est administré 2 fois par semaine, durant 5 semaines à la dose de 250 µg/souris. Un groupe de souris recevant du PBS est introduit comme contrôle étant donné que des expériences effectuées précédemment ont montré que l'administration d'un isotype contrôle IgG1 était sans aucun impact sur la survie des animaux.

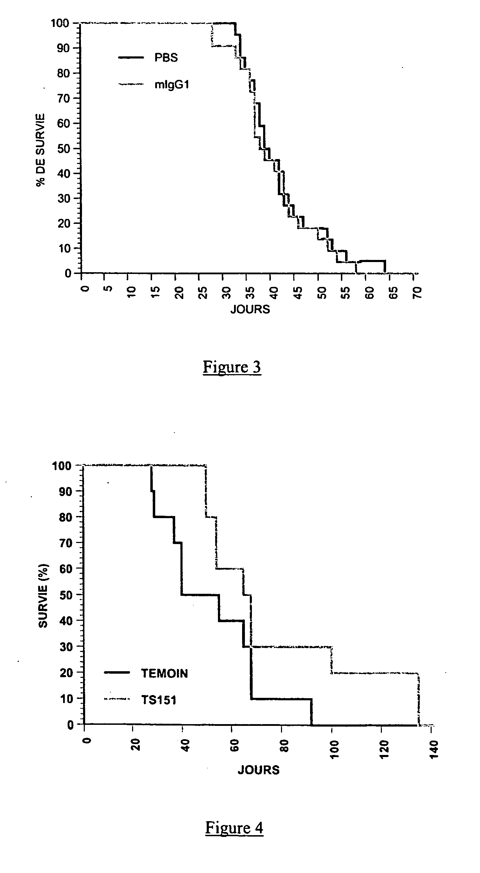

La figure 3, issue de données préliminaires, démontre la spécificité de l'activité observée avec l'anticorps anti-CD 151. En effet, le traitement des animaux avec une IgG 1 murine (mIgG1) utilisée comme isotype contrôle montre que cette dernière n'a aucun impact sur la survie des animaux injecté avec le PBS utilisé comme véhicule de ces anticorps.

Le paramètre d'évaluation de ce modèle est la survie des animaux et l'activité anti-tumorale est exprimée par le calcul du T/C%= médiane de survie des animaux traités/médiane de survie des animaux du groupe control X 100. Il a été établi qu'un T/C% supérieur ou égale à 125 % signe une activité du produit.

### Résultats

La Figure 4 montre une activité antitumorale de l'anticorps TS151 avec un T/C% calculé de 140%

### Exemple 3 : Comparaison de l'activité anti-tumorale in vivo des anticorps TS151 et 50-6 dans un modèle orthotopique A549

### Matériel et Méthode

Le protocole mis en oeuvre est identique à celui de l'exemple 2 ci-dessus.

### Résultats

Les données obtenus mettent clairement en évidence que l'anticorps TS151 présente une activité anti-tumorale nettement supérieure à celle montrée par l'anticorps 50-6 qui ne présente, quant à lui, qu'un T/C% de 118, c'est-à-dire inférieur à la valeur seuil de 125% (données non montrées).

Les résultats obtenus, à savoir le T/C% calculé comme défini plus haut, sont représentés dans le tableau 4 ci-dessous.

**Tableau 4**

| | **Anticorps TS151** | **Anticorps 50-6** |
|---|---|---|
| **T/C%** | 140 | 118 |

### Exemple 4 : Etude de l'expression de la molécule CD151

L'expression de la protéine CD151 a été recherchée par immunohistochimie dans des échantillons de tissus humains issus de patients atteints de cancers de la prostate ou de cancer du poumon. Pour ces patients des lames de tissus normaux adjacents à la tumeur étaient disponibles et ont donc été incluses pour calibrer le niveau d'expression dans les tissus tumoraux *versus* normaux.

Pour ces expériences, des lames de type « Tissue array » commerciales sont utilisées. Après déparaffinage, un démasquage antigénique est effectué à 30°C à l'aide d'une solution enzymatique contenant de la pepsine (Labvision ref. AP-9007-005). Cette étape est suivie par une étape d'élimination des peroxydases endogènes par incubation des coupes dans une solution de peroxyde d'hydrogène (Sigma) à 0,3% dans l'eau. Une saturation des sites non spécifiques est alors effectuée avec une solution d'Ultra-V-Block (Labvision, ref. TA-125-UB) et le marquage est effectué à l'aide d'un anticorps murin anti-CD151 commercial (Serotech, Ref. MCA 1856) utilisé à une concentration finale de 5 µg/ml. Un anticorps isotype contrôle IgG1 murin (DakoCytomation, Ref. X0931) est utilisé comme contrôle négatif d'expérience. La révélation du marquage se fait avec le système de révélation Envision Dual Link (DakoCytomation, Ref. K4061) et la référence de la DAB, substrat des peroxydases est S3309 de DakoCytomation.

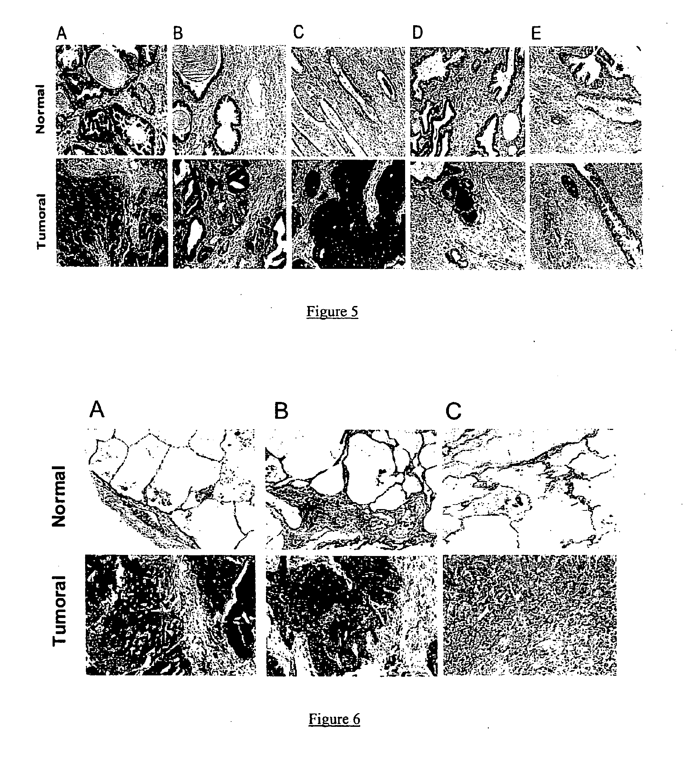

Les résultats présentés dans la figure 5 montrent que plusieurs patients développant des tumeurs de la prostate présentent une sur-expression de la molécule CD 151. Cette sur-expression peut être très significative pour 20% des patients étudiés (patients A et C) ou modérée (patients A et D). Il est à noter que, excepté au niveau des cellules endothéliales, les tissus normaux prostatiques correspondant n'expriment pas ou peu le CD151 et que, en cas d'expression, celle-ci semble être limitée à des structures de type glandulaires. Le patient E présente un exemple de tumeur n'exprimant pas CD151.

Dans le cas du cancer du poumon (Figure 6), une expression modérée (patient A) à forte (patient B) est observée au niveau de certaines cellules du tissu pulmonaire normal. Cependant le tissu tumoral présente une très grande densité de cellules fortement marquées (patients A et B). Le patient C présente un exemple de tumeur n'exprimant pas CD151.

### Exemple 5 : Effet des anticorps TS151 et TS151R sur la croissance in vivo de la tumeur PC3 implantée en sous cutanée chez la souris Nude.

Etant donné les résultats obtenus en immunohistochimie sur les « tissue array » de prostate, une évaluation des anticorps anti-CD 151 sur une xénogreffe de tumeur PC3 a été envisagée. La lignée PC3 est une lignée prostatique androgéno-indépendante originaire de l'ATCC et cultivée en milieu F12K+10% SVF+L-Glutamine. Pour l'évaluation, 5.10⁶ cellules PC3 sont implantées sur le flanc droit de souris Swiss Nude. Cinq jours après implantation, les animaux sont randomisés sur la base du volume tumoral et répartis en 3 groupes comparables. Le volume tumoral du lot d'animaux greffé sélectionné est compris entre 41 et 47 mm³ (volume calculé avec la formule π/6 x Longueur x largeur x épaisseur) au jour 0 du traitement. Les animaux reçoivent alors les anticorps purifiés à tester ou du PBS. Les doses d'anticorps et la fréquence des injections sont les suivantes : dose d'appel 2 mg/dose d'anticorps ; dose de maintien 1 mg/dose 2 fois par semaine.

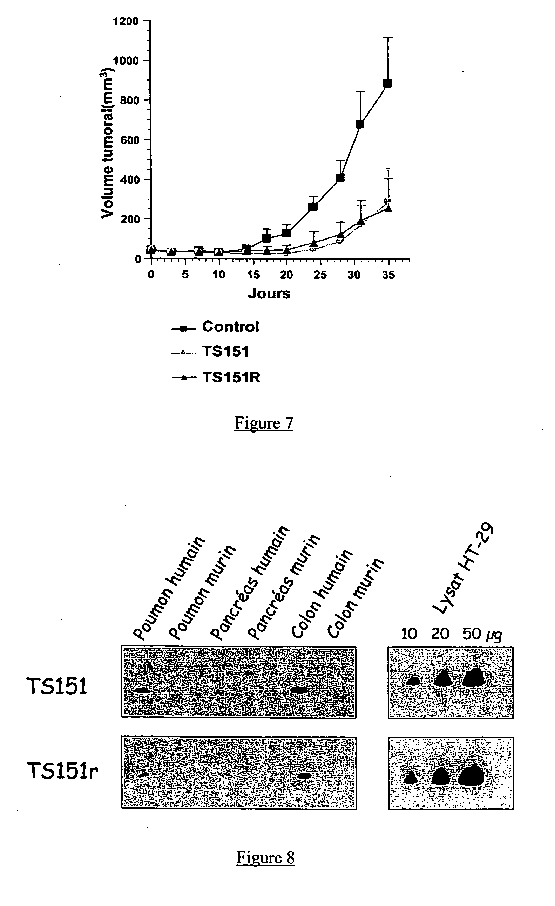

Les résultats présentés en figure 7 montrent que les deux anticorps testés (TS 151 et TS151R) se comportent de façon comparable et inhibent très significativement la croissance de la tumeur PC3 implantée en position sous-cutanée chez la souris Swiss Nude. Le tableau 5 ci-dessous résume les analyses statistiques de ces résultats.

**Tableau 5**

| | | J0 | J3 | J7 | J10 | J14 | J17 | J20 | J24 | J28 | J31 | J35 |
|---|---|---|---|---|---|---|---|---|---|---|---|---|
| control/TS151 | Mann-Whitney (Wilcoxon) | p=0,132 | p=0,937 | p=0.085 | p=0.589 | p=0,002 | p=0,002 | p=0,002 | p=0,002 | p=0,002 | p=0,002 | p=0,002 |
| control/TS151 | Mann-Whitney (Wilcoxon) | p=0,485 | p=0,180 | p=0,180 | p=0,569 | p=0,240 | p=0,26 | p=0,004 | p=0,002 | p=0,002 | p=0,002 | p=0,002 |

Des études menées en parallèle et présentées dans la figure 8 montrent que les anticorps TS 151 et TS151R reconnaissent spécifiquement la molécule CD151 humaine sans aucune réaction croisée avec le récepteur murin. Cette observation suggère donc que l'activité observée dans le modèle de xénogreffe chez la souris nude ne peut être attribuée qu'à un effet direct sur le tissus humain greffé et exclue, par conséquent, toute interférence des anticorps TS151 et TS 151 R avec des cellules du stroma de la tumeur ou des cellules endothéliales murines. Par ailleurs, TS151 et TS151R sont deux IgG1 murine et de ce fait et comme connu par l'homme de l'art, il est peu probable que l'activité observée soit liée à des fonctions effectrices de type ADCC et CDC qui sont plus particulièrement médiées par des anticorps de type IgG2a murine chez la souris.

L'ensemble de ces résultats est donc en accord avec un mécanisme d'action directement lié à l'inhibition de la prolifération cellulaire tumorale *in vivo* par les anticorps TS151 et TS151R.

### Exemple 6 : Spécificité des anticorps TS151 et TS151r

La spécificité des anticorps TS151 et TS151r a été évaluée par western blot. Des lysats de tissus humains et murins de poumon, pancréas et colon (Biochain, 10 µg de protéines totales), ainsi que des quantités croissantes de lysat cellulaire de HT-29 (10, 20 et 50 µg de protéines totales) ont été déposés sur un gel d'acrylamide 4-12 % (BioRad). Après électrophorèse (conditions non réductrices), les protéines ont été transférées sur membrane de nitrocellulose. Les membranes de transfert ont ensuite été incubées avec les anticorps TS151 et TS151r purifiés, puis avec un anticorps polyclonal de lapin anti-Ig de souris couplé à la péroxydase (GE Healthcare) avant révélation de type ECL.

Les anticorps TS151 et TS151r présentent une spécificité pour la forme humaine de CD151 comme en atteste la reconnaissance par western blot de CD151 dans des lysats de cellules HT-29 et de différents tissus d'origine humaine (figure 8). L'absence de réactivité avec le CD 151 murin dans les lysats de différents tissus prélevés chez la souris confirme la spécificité des anticorps TS 151 et TS151r pour la forme humaine de CD 151.

### Exemple 7 : Inhibition de l'adhésion cellulaire

Les expériences d'adhésion de cellules tumorales sur la laminine 5, ligand des intégrines α3β1 et α6β4 avec lesquelles peut s'associer CD151, sont réalisées en plaque 96 puits. Après immobilisation de la laminine 5 (Chemicon, 200 µl à 1 µg/ml) pendant 1 heure à 37°C, les puits sont saturés par de la BSA à 2 mg/ml (200 µl, 1 h à 37°C). Les cellules A549 en suspension sont marquées à l'aide de 5-chlorométhylfluorescéine diacétate (CMFDA, Invitrogen), puis ajoutées à raison de 100000 cellules (100 µl) par puit en présence ou absence d'anticorps (100 µl). Après incubation à 37°C pendant 15, 30 ou 60 minutes, les cellules n'ayant pas adhéré sont éliminées. Après lecture de la chimioluminescence à l'aide d'un luminomètre (Mithras, Berthold), le pourcentage de cellules ayant adhéré est déterminé à l'aide d'une gamme de cellules marquées au CMFDA. L'anticorps anti-CD 151 TS 151 et les anticorps anti-intégrine α3 P1B5, anti-α6 NKI-Go3 et anti-β4 ASC-3 (Chemicon) sont évalués à la concentration finale de 20 µg/ml. L'anticorps 9G4, dirigé contre une protéine de la membrane de *Escherichia coli*, est utilisé comme contrôle isotypique.

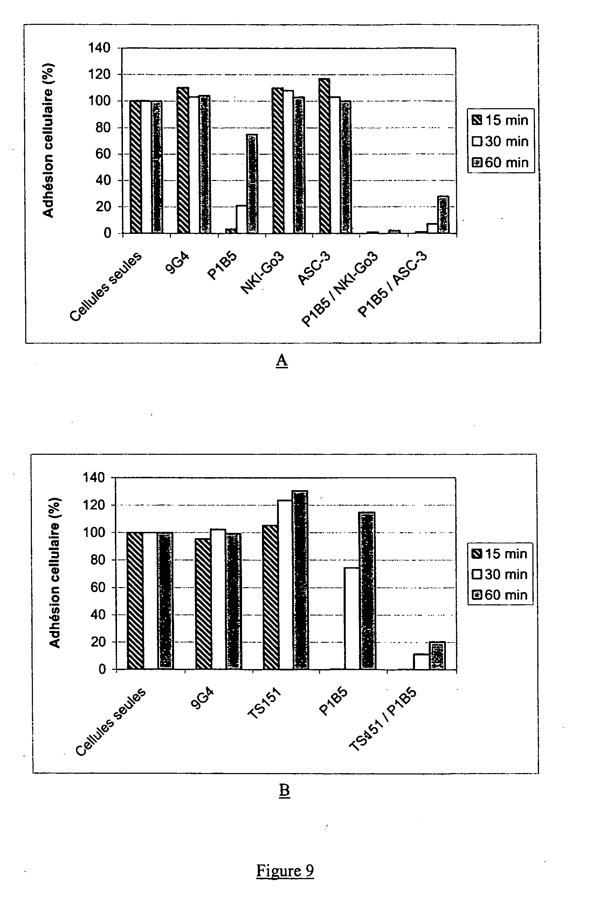

L'anticorps anti-intégrine α3 P1B5 inhibe l'adhésion des cellules A549 sur la laminine 5 (Figure 9A), alors que les anticorps anti-intégrine α6 NKI-Go3 et anti-intégrine β4 ASC-3 n'inhibent pas l'adhésion des cellules A549 sur ce même ligand. On constate toutefois une perte d'inhibition en fonction du temps. L'inhibition induite par le P1B5 est en effet supérieure à 90% à 15 min, mais tombe à environ 20% après 1 h. L'association de l'anticorps P1B5 avec l'anticorps anti-intégrine α6 NKI-Go3 ou l'anticorps anti-intégrine β4 ASC-3 permet de maintenir une forte inhibition d'adhésion après 1 h : celle-ci est supérieure à 90% pour l'association avec l'anticorps anti-□6, et d'environ 70% pour la combinaison avec l'anticors anti-β4. Ces résultats démontrent que les cellules A549 adhèrent sur la laminine 5 dans un premier temps par l'intermédiaire de l'intégrine α3β1, puis dans un deuxième temps par l'intermédiaire de l'intégrine α6β4.

L'anticorps anti-CD151 TS151 n'inhibe pas l'adhésion des cellules A549 sur la laminine 5 lorsqu'il est utilisé seul (Figure 9B). L'effet d'une combinaison du TS151 avec le P1B5 sur l'adhésion des cellules A549 a ensuite été évalué et comparé avec les combinaisons précédemment mentionnées. Cette combinaison TS151/P1B5 donne un résultat comparable à l'association d'anticorps anti-α6/anti-α3 et anti-β4/anti-α3. On observe en effet un maintien de l'inhibition d'adhésion, de l'ordre de 80% après 1 h. L'anticorps TS151 serait donc capable d'inhiber l'adhésion des cellules A549 par l'intermédiaire d'un effet antagoniste sur l'intégrine α6β4.

## Revendications

1. Utilisation d'au moins un anticorps ou l'un de ses fragments fonctionnels, capable de se lier à la protéine CD151 et d'inhiber la croissance tumorale pour la préparation d'un médicament destiné au traitement des tumeurs primaires.

2. Utilisation selon la revendication 1, **caractérisée en ce que** ledit anticorps anti-CD151, ou l'un de ses fragments fonctionnels, est capable d'inhiber la prolifération des cellules tumorales.

3. Utilisation selon la revendication 1 ou 2, **caractérisée en ce que** ledit anticorps anti-CD151, ou l'un de ses fragments fonctionnels, est capable d'inhiber l'activité promotrice de métastase de ladite protéine CD 151 au sein des cellules tumorales.

4. Utilisation selon l'une quelconque des revendications 1 à 3, **caractérisée en ce que** ledit anticorps anti-CD151, ou l'un de ses fragments fonctionnels, est capable d'inhiber l'invasion cellulaire des cellules tumorales.

5. Utilisation selon l'une quelconque des revendications précédentes, **caractérisée en ce que** ledit anticorps anti-CD151, ou l'un de ses fragments fonctionnels, est capable d'inhiber l'adhésion cellulaire des cellules tumorales.

6. Utilisation selon l'une quelconque des revendications précédentes, **caractérisée en ce que** ledit anticorps anti-CD 151, ou l'un de ses fragments fonctionnels, consiste en un anticorps monoclonal.

7. Utilisation selon la revendication 6, **caractérisée en ce que** ledit anticorps comprend :
- les 3 CDRs de la chaîne lourde CDR-H1, CDR-H2 et CDR-H3 respectivement de séquence SEQ ID No. 7, 8 et 9 ; et
- les 3 CDRs de la chaîne légère CDR-L1, CDR-L2 et CDR-L3 respectivement de séquence SEQ ID No. 11, 12 et 13.

8. Utilisation selon la revendication 7, **caractérisée en ce que** ledit anticorps comprend une chaîne lourde comprenant la séquence SEQ ID No. 10 et une chaîne légère comprenant la séquence SEQ ID No. 14.

9. Utilisation selon la revendication 6, **caractérisée en ce que** ledit anticorps comprend :
- les 3 CDRs de la chaîne lourde CDR-H1, CDR-H2 et CDR-H3 respectivement de séquence SEQ ID No. 15, 16 et 17 ; et
- les 3 CDRs de la chaîne légère CDR-L1, CDR-L2 et CDR-L3 respectivement de séquence SEQ ID No. 19, 20 et 21.

10. Utilisation selon la revendication 9, **caractérisée en ce que** ledit anticorps comprend une chaîne lourde comprenant la séquence SEQ ID No. 18 et une chaîne légère comprenant la séquence SEQ ID No. 22.

11. Utilisation selon l'une quelconque des revendications précédentes, **caractérisée en ce que** lesdits cancers consistent en les cancers du colon, du poumon, de la prostate ou du pancréas.

12. Utilisation selon l'une quelconque des revendications précédentes pour la préparation d'une composition pharmaceutique comprenant, en outre, au moins un véhicule pharmaceutiquement acceptable.

13. Composition **caractérisée en ce qu'**elle comprend à titre de principe actif au moins un anticorps anti-CD151, ou l'un de ses fragments fonctionnels, capable de se lier à la protéine CD151 et d'inhiber le développement de tumeurs primaires.

14. Composition selon la revendication 13 pour son utilisation dans le traitement des tumeurs primaires, **caractérisée en ce que** ledit anticorps anti-CD151, ou l'un de ses fragments fonctionnels, est un anticorps monoclonal sélectionné parmi les anticorps
- TS151 comprenant les 3 CDRs de la chaîne lourde CDR-H1, CDR-H2 et CDR-H3 respectivement de séquence SEQ ID No. 7, 8 et 9 ; et les 3 CDRs de la chaîne légère CDR-L1, CDR-L2 et CDR-L3 respectivement de séquence SEQ ID No. 11, 12 et 13 ; ou
- TS151r comprenant les 3 CDRs de la chaîne lourde CDR-H 1, CDR-H2 et CDR-H3 respectivement de séquence SEQ ID No. 15, 16 et 17 ; et les 3 CDRs de la chaîne légère CDR-L1, CDR-L2 et CDR-L3 respectivement de séquence SEQ ID No. 19, 20 et 21.

15. Composition selon la revendication 13 ou 14 pour son utilisation dans le traitement des tumeurs primaires, **caractérisée en ce qu'**elle comprend, en outre, comme produit de combinaison pour une utilisation simultanée, séparée ou étalée dans le temps, au moins un agent cytotoxique/cytostatique et/ou une toxine cellulaire et/ou un radioélément et/ou un anticorps monoclonal.

## Claims

1. Use of at least one antibody or a functional fragment thereof, capable of binding to the D151 protein and of inhibiting tumour growth, for the preparation of a medicament intended for the treatment of primary tumours.

2. Use according to Claim 1, **characterized in that** said anti-CD151 antibody, or a functional fragment thereof, is capable of inhibiting tumour cell proliferation.

3. Use according to Claim 1 or 2, **characterized in that** said anti-CD151 antibody, or a functional fragment thereof, is capable of inhibiting the metastasis-promoting activity of said CD151 protein in tumour cells.

4. Use according to any one of Claims 1 to 3, **characterized in that** said anti-CD151 antibody, or a functional fragment thereof, is capable of inhibiting the cell invasion of tumour cells.

5. Use according to any one of the preceding claims, **characterized in that** said anti-CD151 antibody, or a functional fragment thereof, is capable of inhibiting the cell adhesion of tumour cells.

6. Use according to any one of the preceding claims, **characterized in that** said anti-CD151 antibody, or a functional fragment thereof, consists of a monoclonal antibody.

7. Use according to Claim 6, **characterized in that** said antibody comprises:
- the 3 heavy-chain CDRs CDR-H1, CDR-H2 and CDR-H3, respectively of sequence SEQ ID Nos. 7, 8 and 9; and
- the 3 light-chain CDRs CDR-L1, CDR-L2 and CDR-L3, respectively of sequence SEQ ID Nos. 11, 12 and 13.

8. Use according to Claim 7, **characterized in that** said antibody comprises a heavy chain comprising the sequence SEQ ID No. 10 and a light chain comprising the sequence SEQ ID No. 14.

9. Use according to Claim 6, **characterized in that** said antibody comprises:
- the 3 heavy-chain CDRs CDR-H1, CDR-H2 and CDR-H3, respectively of sequence SEQ ID Nos. 15, 16 and 17; and
- the 3 light-chain CDRs CDR-L1, CDR-L2 and CDR-L3, respectively of sequence SEQ ID Nos. 19, 20 and 21.

10. Use according to Claim 9, **characterized in that** said antibody comprises, a heavy chain comprising the sequence SEQ ID No. 18 and a light chain comprising the sequence SEQ ID No. 22.

11. Use according to any one of the preceding claims, **characterized in that** said cancers consist of colon cancer, lung cancer, prostate cancer or pancreatic cancer.

12. Use according to any one of the preceding claims, for the preparation of a pharmaceutical composition comprising, in addition, at least one pharmaceutically acceptable vehicle.

13. Composition, **characterized in that** it comprises, as active ingredient, at least one anti-CD151 antibody or a functional fragment thereof, capable of binding to the CD151 protein and of inhibiting the development of primary tumours, for use thereof in the treatment of primary tumours.

14. Composition according to Claim 13 for use thereof in the treatment of primary tumours, **characterized in that** said anti-CD151 antibody, or a functional fragment thereof, is a monoclonal antibody selected from the antibodies
- TS151 comprising the 3 heavy-chain CDRs CDR-H1, CDR-H2 and CDR-H3, respectively of sequence SEQ ID Nos. 7, 8 and 9; and the three light-chain CDRs CDR-L1, CDR-L2 and CDR-L3, respectively of sequence SEQ ID Nos. 10, 11 and 12; or
- TS151r comprising the 3 heavy-chain CDRs CDR-H1, CDR-H2 and CDR-H3, respectively of sequence SEQ ID Nos. 15, 16 and 17; and the three light-chain CDRs CDR-L1, CDR-L2 and CDR-L3, respectively of sequence SEQ ID Nos. 19, 20 and 21.

15. Composition according to Claim 13 or 14 for use thereof in the treatment of primary tumours, **characterized in that** it comprises, in addition, as combination product for use simultaneously, separately or sequentially over time, at least one cytotoxic/cytostatic agent and/or one cell toxin and/or one radio element and/or one monoclonal antibody.

## Patentansprüche

1. Verwendung wenigstens eines Antikörpers, oder eines seiner funktionellen Fragmente, welcher dazu in der Lage ist, an das Protein CD151 zu binden und das Tumorwachstum zu hemmen, zur Herstellung eines Arzneimittels, das zur Behandlung von Primärtumoren vorgesehen ist.

2. Verwendung nach Anspruch 1, **dadurch gekennzeichnet, dass** der Anti-CD151-Antikörper, oder eines seiner funktionellen Fragmente, dazu in der Lage ist, die Proliferation von Tumorzellen zu hemmen.

3. Verwendung nach Anspruch 1 oder 2, **dadurch gekennzeichnet, dass** der Anit-CD151-Antikörper, oder eines seiner funktionellen Fragmente, dazu in der Lage ist, die Metastasenbildung begünstigende Aktivität des Proteins CD151 in den Tumorzellen zu hemmen.

4. Verwendung nach einem der Ansprüche 1 bis 3, **dadurch gekennzeichnet, dass** der Anti-CD151-Antikörper, oder eines seiner funktionellen Fragmente, dazu in der Lage ist, eine Zellinvasion der Tumorzellen zu hemmen.

5. Verwendung nach einem der vorangegangenen Ansprüche, **dadurch gekennzeichnet, dass** der Anti-CD151-Antikörper, oder eines seiner funktionellen Fragmente, dazu in der Lage ist, die Zelladhäsion der Tumorzellen zu hemmen.

6. Verwendung nach einem der vorangegangenen Ansprüche, **dadurch gekennzeichnet, dass** der Anti-CD151-Antikörper, oder eines seiner funktionellen Fragmente, aus einem monoklonalen Antikörper besteht.

7. Verwendung nach Anspruch 6, **dadurch gekennzeichnet, dass** der Antikörper umfasst:
- die 3 Antigenbindungsstellen (Complementarity Determining Region, CDR) der schweren Kette CDR-H1, CDR-H2 und CDR-H3, die entsprechend die Sequenz SEQ ID NO: 7, 8 und 9 aufweisen; und
- die 3 Antigenbindungsstellen (CDR) der leichten Kette CDR-L1, CDR-L2 und CDR-L3, die entsprechend die Sequenz SEQ ID NO: 11, 12 und 13 aufweisen.

8. Verwendung nach Anspruch 7, **dadurch gekennzeichnet, dass** der Antikörper eine schwere Kette, die die Sequenz SEQ ID NO: 10 aufweist, und eine leichte Kette, die die Sequenz SEQ ID NO: 14 aufweist, umfasst.

9. Verwendung nach Anspruch 6, **dadurch gekennzeichnet, dass** der Antikörper umfasst:
- die 3 Antigenbindungsstellen (CDR) der schweren Kette CDR-H1, CDR-H2 und CDR-H3, die entsprechend die Sequenz SEQ ID NO: 15, 16 und 17 aufweisen; und
- die 3 Antigenbindungsstellen (CDR) der leichten Kette CDR-L1, CDR-L2 und CDR-L3, die entsprechend die Sequenz SEQ ID NO: 19, 20 und 21 aufweisen.

10. Verwendung nach Anspruch 9, **dadurch gekennzeichnet, dass** der Antikörper eine schwere Kette, die die Sequenz SEQ ID NO: 18 aufweist, und eine leichte Kette, die die Sequenz SEQ ID NO: 22 aufweist, umfasst.

11. Verwendung nach einem der vorangegangenen Ansprüche, **dadurch gekennzeichnet, dass** der Krebs Darmkrebs, Lungenkrebs, Prostatakrebs oder Pankreaskrebs ist.

12. Verwendung nach einem der vorangegangenen Ansprüche zur Herstellung einer pharmazeutischen Zusammensetzung, die unter anderem wenigstens einen pharmazeutisch unbedenklichen Träger enthält.

13. Zusammensetzung, **dadurch gekennzeichnet, dass** sie als Wirkstoff wenigstens einen Anti-CD151-Antikörper, oder eines seiner funktionellen Fragmente, enthält, welcher dazu in der Lage ist, an das Protein CD151 zu binden und die Entwicklung von Primärtumoren zu hemmen, zur Verwendung bei der Behandlung von Primärtumoren.

14. Zusammensetzung nach Anspruch 13 zur Verwendung bei der Behandlung von Primärtumoren, **dadurch gekennzeichnet, dass** der Anti-CD151-Antikörper, oder eines seiner funktionellen Fragmente, ein monoklonaler Antikörper ist, der ausgewählt ist aus den Antikörpern
- TS151, der die 3 Antigenbindungsstellen (CDR) der schweren Kette CDR-H1, CDR-H2 und CDR-H3, die entsprechend die Sequenz SEQ ID NO: 7, 8 und 9 aufweisen; und die 3 Antigenbindungsstellen (CDR) der leichten Kette CDR-L1, CDR-L2 und CDR-L3, die entsprechend die Sequenz SEQ ID NO: 11, 12 und 13 aufweisen, umfasst; oder
- TS151r, der die 3 Antigenbindungsstellen (CDR) der schweren Kette CDR-H1, CDR-H2 und CDR-H3, die entsprechend die Sequenz SEQ ID NO: 15, 16 und 17 aufweisen; und die 3 Antigenbindungsstellen (CDR) der leichten Kette CDR-L1, CDR-L2 und CDR-L3, die entsprechend die Sequenz SEQ ID NO: 19, 20 und 21 aufweisen, umfasst.

15. Zusammensetzung nach Anspruch 13 oder 14 zur Verwendung bei der Behandlung von Primärtumoren, **dadurch gekennzeichnet, dass** sie unter anderem als Kombinationsprodukt zur gleichzeitigen, separaten oder über die Zeit verteilten Verwendung wenigstens ein zytotoxisches/zytostatisches Mittel und/oder ein Zellgift und/oder ein Radioelement und/oder einen monoklonalen Antikörper enthält.
